(19) Europäisches Patentamt
European Patent Office
Office européen des brevets

(11)  EP 2 510 359 B1

(12)  **EUROPEAN PATENT SPECIFICATION**

(45) Date of publication and mention
of the grant of the patent:
**02.09.2015   Bulletin 2015/36**

(51) Int Cl.:
***G01N 33/68*** [(2006.01)]

(21) Application number: **10787797.9**

(86) International application number:
**PCT/EP2010/069474**

(22) Date of filing: **13.12.2010**

(87) International publication number:
**WO 2011/070174 (16.06.2011 Gazette 2011/24)**

(54)  **METHODS AND REAGENTS FOR IMPROVED DETECTION OF AMYLOID BETA PEPTIDES**

VERFAHREN UND REAGENZIEN FÜR VERBESSERTEN NACHWEIS VON AMYLOID-BETA-PEPTIDEN

PROCÉDÉS ET RÉACTIFS POUR AMÉLIORER LA DÉTECTION DE PEPTIDES AMYLOÏDES BÊTAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA**

(30) Priority: **11.12.2009   EP 09382279**

(43) Date of publication of application:
**17.10.2012   Bulletin 2012/42**

(73) Proprietor: **Araclón Biotech, S. L.**
**50009 Zaragoza(Zaragoza) (ES)**

(72) Inventor: **SARASA BARRIO, José Manuel**
**E-50004 Zaragoza (ES)**

(74) Representative: **Durán-Corretjer, S.L.P.**
**Còrsega, 329**
**(Paseo de Gracia/Diagonal)**
**08037 Barcelona (ES)**

(56) References cited:
**WO-A1-2004/104597      WO-A1-2009/048631**

• **STEINERMAN JOSHUA R ET AL: "Distinct pools of beta-amyloid in Alzheimer disease-affected brain: a clinicopathologic study.", ARCHIVES OF NEUROLOGY JUL 2008, vol. 65, no. 7, July 2008 (2008-07), pages 906-912, XP002574070, ISSN: 1538-3687**

**Description**

**TECHNICAL FIELD**

The invention relates to the field of immunoassays and, more specifically, to the methods for increasing the sensitivity of immunoassays for the determination of amyloid beta peptides in biological fluids.

**BACKGROUND OF THE INVENTION**

Alzheimer's disease (AD) is a progressive degenerative disease of the central nervous system characterized by progressive and increasing memory loss, followed by loss of control of limbs and bodily functions and eventual death. It is by far the most common cause of dementia affecting 1 to 6% of people over the age of 65 years and between 10 to 20% of those over 80.

AD is distinguished from other types of dementia by several pathological features, including the progressive appearance in the brain of the patients of senile plaques in the extracellular space between neurons. The plaques have central cores of amyloid deposits formed mainly by fibrils of a 40-42 amino acids peptide referred to amyloid $\beta$ peptide (A$\beta$) surrounded by degenerated neuritis and glial cells. This peptide results from the proteolytic processing of a precursor protein called $\beta$ amyloid precursor protein ($\beta$APP).

AD can be classified according to the age of appearance as early onset (age under 60 years) and late onset (age above 60 years), according to the existence of an autosomic dominant inheritance, as familiar AD or sporadic AD. Early onset familiar forms of AD can be associated to known mutation in the genes coding for $\beta$APP, presenilin 1 and presenilin 2 (located, respectively, on chromosomes 21, 14 and 1). These classifications are not mutually exclusive. The most frequent forms are sporadic late-onset forms.

In clinical praxis, diagnosis of AD is carried out using clinical criteria based on the presence of typical clinical hallmarks and the exclusion of other types of dementia using neuroimaging techniques and blood analysis. Using these criteria, diagnostic reliability is acceptable although, according to studies done using brain autopsy, between 10-20% of the patients diagnosed with AD suffered from a different disease. Moreover, the current diagnostic methods can only be carried out when the neurodegenerative process is so advanced that the patient suffers from severe dementia and the brain damages are so extensive that the number of therapeutic measures is limited. Definitive diagnosis requires pathologic examination of post-mortem brain tissue.

In view of the fact that A$\beta$ accumulates in the brain of AD patients and is a central element in the pathogenesis of AD, this protein has been considered as the most suitable candidate as AD biomarker. However, the use of A$\beta$ as plasma biomarker for AD faces the problem that the concentrations of the A$\beta$ peptides (A$\beta$(1-40) and A$\beta$(1-42)) in serum are extremely low, so that there are no assays which are sensitive enough so as to allow reliable detection of said peptide species.

Many different assays have been used to determine levels of amyloid beta peptides in biological samples (see e.g. the methods described by Scheuner et al (Nature Med., 1996, 2:864-870); Tamaoka A et al. (J Neurol Sci., 1996, 141, 65-68); Suzuki,N. et al. (Science, 1994, 264:1336-1340); WO200722015, Vanderstichele H et al. (Amyloid, 2000, 7, 245-258); Fukomoto y col. (Arch. Neurol. 2003, 60, 958-964); Mehta et al. (Arch. Neurol. 57, 2000, 100-105); Mayeux, R. et al. (Ann Neurol. 1999, 46, 412-416); Lanz, T.A and Schacthter, J.B. (J. Neuroscience Methods, 2006, 157:71-81), WO200750359, WO0162801, WO0315617, WO0246237, WO0413172. However, all the ELISA-based assays known to date have a lower detection limit which is not in the range of single digit pg/mL at the most, which is sufficient for detecting A$\beta$40 and A$\beta$42 in CSF as well as for detecting said species in plasma in patients suffering from familiar AD, but are unsuitable for detecting A$\beta$42 in the plasma of patients suffering from sporadic AD, wherein the A$\beta$42 plasma concentration are much lower.

To date, the only A$\beta$ peptide assays showing a lower detection limit lower than the single digit pg/mL correspond to the assays described in WO200646644 and in WO2009015696.

WO200646644 describes an electrochemiluminiscent (ECL) sandwich assay wherein the mAb 21F12 (which recognises amino acids 33-42 of A$\beta$42) is coupled to magnetic beads, which are then used to capture the A$\beta$42 peptide in the sample containing A$\beta$42 and further contacted with 3D6 mAb coupled to a ruthenium complex. The amount of 3D6 antibody bound is then detected by the luminescence emitted by the ruthenium complex when electrical energy is applied. Using this assay, the inventors are capable of detecting as low as 0.5 pg/mL of a A$\beta$42 standard. However, when the same assay is used to compare A$\beta$42 in plasma samples from AD patients and healthy controls, no significant differences could be observed between the two sets of patients, which led the inventors to conclude that the amount of intact A$\beta$42 in serum is very low due to degradation and turned to a competitive ELISA assay using 21F12 mAb which provides lower sensitivity levels in the range of ng/mL.

WO2009015696 describes a high-sensitivity ELISA sandwich assay wherein the detection antibody is contacted with a biotin-labeled reagent showing specificity for said antibody. The reagent is contacted with streptavidin which is coupled to peroxidase. Peroxidase activity is then detected by colorimetry using TMB or fluorescently using QuantaBlue.

WO2006053251 describes a method for the determination of amyloid beta peptide species in a sample comprising contacting a sample with a denaturing agent, extracting the peptide pool from the sample-denaturing agent mixture, separating the amyloid beta peptide species from the pool and determining the amount of amyloid beta peptide species. This method requires a step of separation of the peptides prior to the determination, which results in increased processing time and increased costs.

Therefore, there is a need in the art for improved immunological assays and kits to detect Aβ-derived peptides which overcome the problems of the methods and kits known in the art, in particular, which are sensitive enough to detect Aβ peptides in a reliable manner in plasma of patients suffering from sporadic AD.

SUMMARY OF THE INVENTION

In a first aspect, the invention relates to a method for the diagnosis in a subject of a neurodegenerative disease, for detecting a stage prior to a neurodegenerative disease or for distinguishing a neurodegenerative disease from a stage prior to said neurodegenerative disease comprising the steps of

(i) determining one or more parameters selected from the group of

(a) the level of one or more free amyloid beta peptides in a biological sample of said subject,
(b) the aggregate levels of a one or more free amyloid peptides in a biological sample of said subject and of said one or more amyloid beta peptides associated to macromolecular components present in said biological sample, wherein said aggregate levels are determined by quantifying the amount of said one or more amyloid beta peptides in cell-free fraction of said sample after contacting said sample with a protein solubilising agent under conditions adequate to promote dissociation of the amyloid beta peptide or peptides from the components present in the biological sample,
(c) the level of one or more amyloid beta peptides associated to cells in a biological sample of said subject, wherein said level is determined by isolating the cell fraction of said biological sample, contacting said cellular fraction of said sample with a protein solubilising agent under conditions adequate to promote dissociation of the amyloid beta peptide or peptides from the cells present in the sample

(ii) comparing the value of at least one of the parameters (b) or (c) or the value of a calculated parameter resulting from arithmetically combining at least two of the parameters (a) to (c) with a reference value corresponding to the value of said parameters (b) or (c) or said calculated parameter in a reference sample and

(iii) diagnosing the neurodegenerative disease, detecting a stage prior to a neurodegenerative disease or distinguishing a neurodegenerative disease from a stage prior to said neurodegenerative disease when there is an alteration in the value of the parameter or in the value of the calculated parameter with respect to the reference value.

wherein the biological sample is plasma or blood, and wherein the parameters determined in step (i) are one or more of the parameters selected from the group of

(a) 1ab40, corresponding to the level of free ABETA40 peptide in a biological sample of said subject,
(b) 1ab42, corresponding to the level of free ABETA42 peptide in a biological sample of said subject,
(c) 2ab40, corresponding to the aggregate levels of free ABETA40 peptide in a biological sample of said subject and of ABETA40 peptide associated to components of said biological sample, wherein 2ab40 is determined by quantifying the amount of ABETA40 peptide in said sample after contacting said sample with a protein solubilising agent under conditions adequate to promote dissociation of the ABETA40 peptide from the components present in the biological sample,
(d) 2ab42, corresponding to the aggregate level of free ABETA42 peptide in a biological sample of said subject and of ABETA42 peptide associated to components of said biological sample, wherein 2ab42 is determined by quantifying the amount of ABETA42 peptide in said sample after contacting said sample with a protein solubilising agent under conditions adequate to promote dissociation of the ABETA42 peptide from the components present in the biological sample,
(e) 3ab40, corresponding to the level of the ABETA40 peptide associated to cells in a biological sample of said subject, wherein 3ab40 is determined by quantifying the amount of ABETA40 peptide after contacting the cellular fraction of said biological sample with a protein solubilising agent under conditions adequate to promote dissociation of the amyloid beta peptides from the cells present in the sample and
(f) 3ab42, corresponding to the level of the ABETA42 peptide associated to cells in a biological sample of said subject, wherein 3ab42 is determined by quantifying the amount of ABETA42 peptide after contacting the cellular fraction of said biological sample with a protein solubilising agent under conditions adequate to promote dissociation of the amyloid beta peptides from the cells present in the sample.

In a second aspect, the invention relates to a kit for determination of amyloid beta peptides in a biological sample comprising

(i) a protein solubilising agent and
(ii) at least an antibody against a amyloid beta peptide.

BRIEF DESCRIPTION OF THE FIGURES

Figure 1: A-F. Dot-plots of the measurements for (A) UP Aβ1-40, (B) DP Aβ1-40, (C) CBAβ1-40, (D) UP Aβ1-42, (E) DP Aβ1-42 and (F) CB Aβ1-42 obtained in the two external laboratories (Lab1 and Lab2). Most points are close to the concordance line indicating a substantial to almost perfect degree of agreement in the measurements. Bottom right insert in each plot indicates the concordance correlation coefficient (CCC) and the 95 % confidence intervals.

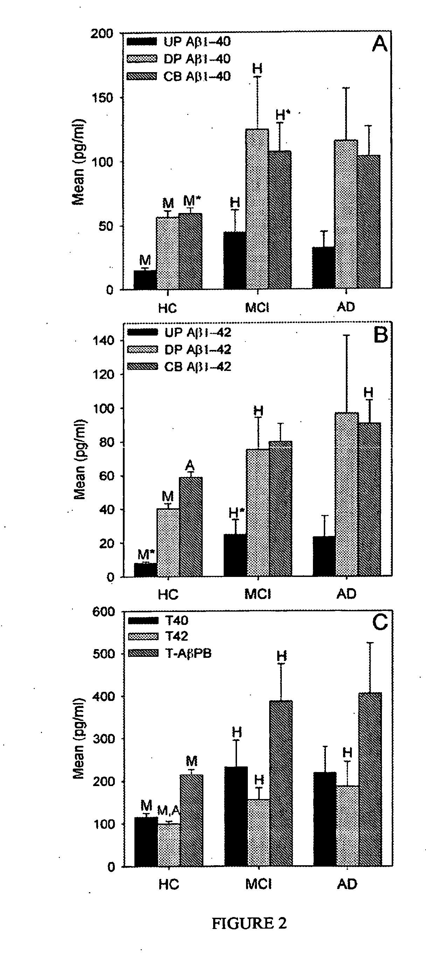

Figure 2: Direct markers of Aβ1-40 (A), of Aβ1-42 (B) and calculated markers of Aβ1-40 and Aβ1-42 (C). A. Concentrations in pg/ml of Aβ1-40 free in serum (FP), total Aβ1-40 levels in plasma (including free Aβ1-40 and Aβ1-40 bound to plasma components) (TP) and Aβ1-40 bound to cells (CB) in healthy controls (HC), patients suffering mild cognitive impairment (MCI) and patients suffering Alzheimer's disease (AD). **B**. Concentrations in pg/ml of Aβ1-42 free in serum (FP), total Aβ1-42 levels in plasma (including free Aβ1-42 and Aβ1-42 bound to plasma components) (TP) and Aβ1-42 bound to cells (CB) in healthy controls (HC), patients suffering mild cognitive impairment (MCI) and patients suffering Alzheimer's disease (AD). C. Aggregated values in pg/ml of total plasma Aβ1-40 (obtained by contacting a plasma sample with a protein solubilising agent) plus cell-bound Aβ1-40 (TP+CB Aβ1-40), of total plasma Aβ1-42 (obtained by contacting a plasma sample with a protein solubilising agent) plus cell-bound Aβ1-42 (TP+CB Aβ1-42), or the aggregated values of TP+CB Aβ1-40 and Aβ1-42 (TP+CB Aβ1-42). H, M, and A mean significant ($p < 0.05$) with regard to HC, MCI and AD, respectively. * means $p < 0.01$.

Figure 3: A-F. Dot-plot for (A) DP Aβ1-40, (B) CB Aβ1-40, (C) UP Aβ1-42, (D) DP Aβ1-42, (E) T40 and (F) T-βAPB values in HC, MCI and AD participants. Numbers beside *indicates the value for outliers in the MCI and AD groups which, for the clarity of the representation, are not represented at the same scale of the ordinate axis. Horizontal line represents the cutoff value between MCI and HC.

Figure 4: ROC curve for the 1ab40 marker in patients with MCI/HC. Area under the ROC curve=0.510.

Figure 5: ROC curve for the 2ab40 marker in patients with MCI/HC. Area under the ROC curve=0.778.

Figure 6: ROC curve for the 3ab40 marker for patients with MCI/HC. Area under the ROC curve=0.458.

Figure 7: ROC curve for the 1ab42 marker for patients with MCI/HC. Area under the ROC curve=0.576

Figure 8: ROC curve for the 2ab42 marker for patients with MCI/HC. Area under the ROC curve=0.667

Figure 9: ROC curve for the 3ab42 marker for patients with AD/HC. Area under the ROC curve=0.744

Figure 10: ROC curve for the 3ab42 marker for patients with MCI/HC. Area under the ROC curve=0.508

Figure 11: ROC curve for the 2ab40 + 3ab40 marker for patients with MCI/HC. Area under the curve is 0.830.

Figure 12: ROC curve for the 2ab42 + 3ab42 marker for patients with AD/HC. Area under the curve is 0.713.

Figure 13: ROC curve for the 2ab42 + 3ab42 marker for patients with MCI/HC. Area under the curve is 0.777.

Figure 14: ROC curve for the 2ab40 + 3ab40 + 2ab42 + 3ab42 marker with MCI/HC. Area under the curve is 0.848.

DETAILED DESCRIPTION OF THE INVENTION

The authors of the present invention have found that, surprisingly, the dilution of the plasma with sample buffer results in an increase in the detectable levels of $A\beta1\text{-}40$ and $A\beta1\text{-}42$. Without wishing to be bound by any theory, it is believed that the dilution of the plasma results in a change in the ionic strength and in the molecular interactions within the sample leading to the release of $A\beta1\text{-}40$ and $A\beta1\text{-}42$ bound to plasma proteins and other components.

Thus, the increment in the measurements after dilution of the plasma might be due to the detection of $A\beta$ peptides released from proteins and other plasma components and could be interpreted as an estimation of the total level of $A\beta$ in plasma.

In any case, these results show that $A\beta$ peptide levels in blood are much higher than it could be estimated from simply assaying their levels in undiluted plasma. A complete determination of the total $\beta$APB blood levels should include the quantification of the peptides free in plasma, bound to plasma proteins and bound to blood cells. This comprehensive quantification of the different components of the $\beta$APB would give a more precise measure of $A\beta$ blood levels and might help to ascertain the complex regulation of $A\beta$ peptides in health and disease. Moreover, the levels of said amyloid beta peptides pools as well as the value of certain calculated parameters resulting from arithmetically combining the concentrations of the different pools with the concentrations of free amyloid beta peptides can be used for determining whether a patient suffers a neurodegenerative disease, whether a patient suffers a state prior to a neurodegenerative disease and to distinguish a neurodegenerative disease from a state prior to a neurodegenerative disease.

Diagnostic method of the invention

Thus, in a first aspect, the invention relates to a method for the diagnosis in a subject of a neurodegenerative disease, for detecting a stage prior to a neurodegenerative disease or for distinguishing a neurodegenerative disease from a stage prior to said neurodegenerative disease comprising the steps of

(i) determining one or more parameters selected from the group of

(a) the level of one or more free amyloid beta peptides in a biological sample of said subject,
(b) the aggregate levels of a one or more free amyloid peptides in a biological sample of said subject and of said one or more amyloid beta peptides associated to macromolecular components present in said biological sample, wherein said aggregate levels are determined by quantifying the amount of said one or more amyloid beta peptides in cell-free fraction of said sample after contacting said sample with a protein solubilising agent under conditions adequate to promote dissociation of the amyloid beta peptide or peptides from the components present in the biological sample,
(c) the level of one or more amyloid beta peptides associated to cells in a biological sample of said subject, wherein said level is determined by isolating the cell fraction of said biological sample, contacting said cellular fraction of said sample with a protein solubilising agent under conditions adequate to promote dissociation of the amyloid beta peptide or peptides from the cells present in the sample and

(ii) comparing the value of at least one of the parameters (b) or (c) or the value of a calculated parameter resulting from arithmetically combining at least two of the parameters (a) to (c) with a reference value corresponding to the value of said parameters (b) or (c) or said calculated parameter in a reference sample and
(iii) diagnosing the neurodegenerative disease, detecting a stage prior to a neurodegenerative disease or distinguishing a neurodegenerative disease from a stage prior to said neurodegenerative disease when there is an alteration in the value of the parameter or in the value of the calculated parameter with respect to the reference value.

wherein the biological sample is plasma or blood, and wherein the parameters determined in step (i) are one or more of the parameters selected from the group of

(a) 1ab40, corresponding to the level of free ABETA40 peptide in a biological sample of said subject,
(b) 1ab42, corresponding to the level of free ABETA42 peptide in a biological sample of said subject,
(c) 2ab40, corresponding to the aggregate levels of free ABETA40 peptide in a biological sample of said subject and of ABETA40 peptide associated to components of said biological sample, wherein 2ab40 is determined by quantifying the amount of ABETA40 peptide in said sample after contacting said sample with a protein solubilising agent under conditions adequate to promote dissociation of the ABETA40 peptide from the components present in the biological sample,
(d) 2ab42, corresponding to the aggregate level of free ABETA42 peptide in a biological sample of said subject and of ABETA42 peptide associated to components of said biological sample, wherein 2ab42 is determined by quantifying the amount of ABETA42 peptide in said sample after contacting said sample with a protein solubilising agent under conditions adequate to promote dissociation of the ABETA42 peptide from the components present in the biological sample,

(e) 3ab40, corresponding to the level of the ABETA40 peptide associated to cells in a biological sample of said subject, wherein 3ab40 is determined by quantifying the amount of ABETA40 peptide after contacting the cellular fraction of said biological sample with a protein solubilising agent under conditions adequate to promote dissociation of the amyloid beta peptides from the cells present in the sample and

(f) 3ab42, corresponding to the level of the ABETA42 peptide associated to cells in a biological sample of said subject, wherein 3ab42 is determined by quantifying the amount of ABETA42 peptide after contacting the cellular fraction of said biological sample with a protein solubilising agent under conditions adequate to promote dissociation of the amyloid beta peptides from the cells present in the sample.

The term "diagnosis" as used herein includes the assessment of a subject's susceptibility to a disease, determination as to whether a subject presently has the disease, and also the prognosis of a subject affected by the disease. As will be understood by persons skilled in the art, such assessment normally may not be correct for 100% of the subjects to be diagnosed, although it preferably is correct. The term, however, requires that a statistically significant part of the subjects can be identified as suffering from the disease or having a predisposition thereto. If a part is statistically significant it can be determined simply by the person skilled in the art using several well known statistical evaluation tools, for example, determination of confidence intervals, determination of p values, Student's t-test, Mann-Whitney test, etc. Details are provided in Dowdy and Wearden, Statistics for Research, John Wiley & Sons, New York 1983. The preferred confidence intervals are at least 50%, at least 60%, at least 70%, at least 80%, at least 90%, at least 95%. The p values are preferably 0.2, 0.1 or 0.05.

As used herein, the term "subject" relates to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race.

The term "neurodegenerative disease", as used herein, refers to a condition or disorder in which neuronal cells are lost due to cell death bringing about a deterioration of cognitive functions or result in damage, dysfunction, or complications that may be characterized by neurological, neurodegenerative, physiological, psychological, or behavioral aberrations. Suitable neurodegenerative diseases that can be diagnosed with the methods of the invention include, without limitation, age-related macular degeneration, Creutzfeldt-Jakob disease, Alzheimer's Disease, radiotherapy induced dementia, axon injury, acute cortical spreading depression, alpha-synucleinopathies, brain ischemia, Huntington's disease, permanent focal cerebral ischemia, peripheral nerve regeneration, post-status epilepticus model, spinal cord injury, sporadic amyotrophic lateral sclerosis and transmissible spongiform encephalopathy.

In a preferred embodiment, the neurodegenerative disease is Alzheimer's disease. The term "Alzheimer's Disease" (or "senile dementia") refers to a mental deterioration associated with specific degenerative brain disease that is characterized by senile plaques, neuritic tangles, and progressive neuronal loss which manifests clinically in progressive memory deficits, confusion, behavioral problems, inability to care for oneself, gradual physical deterioration and, ultimately, death. Patients suffering Alzheimer's disease are identified using the NINCDS-ADRDA criteria (CDR = 1, MMSE between 16 and 24 points and Medial temporal atrophy (determined by MRI) >3 points in Scheltens scale.

The term "stage prior to said neurodegenerative disease", as used herein, refer to a transitional situation which occurs between normal individuals and subjects suffering from a neurodegenerative disorders and which is characterized by the appearance of some of the signs and symptoms of the neurodegenerative disorders or by the appearance of a subset of the sign and symptoms observed in patients suffering the neurodegenerative disorder. In a preferred embodiment, the stage prior to said neurodegenerative disease is mild cognitive impairment (hereinafter MCI) which refers to a transitional stage of cognitive impairment between normal aging and early Alzheimer's disease. Patients are usually identified as having MCI if they fulfill the Mayo Clinic criteria (CDR = 0.5, they show a medial temporal atrophy (determined by MRI) which is higher than 3 points in Scheltens scale, they show a pattern of parietal and/or temporal hypometabolism in Positron Emission Tomography with 18-fluorodeoxyglucose (PET-FDG) (suggestive of AD).

The term "distinguishing a neurodegenerative disease from a stage prior to said neurodegenerative disease" refers to the capability of discriminating between a patient found in the prior or prodromic stage of a neurodegenerative disease from patients which are already suffering the disease. In the particular case that the neurodegenerative disease is Alzheimer's disease, the method of the invention allows the distinguishing between Alzheimer's disease and the prodromic stage of said disease known as mild cognitive impairment (MCI):

In a first step, the method of the invention comprises the determination of at least one parameter selected from the group of

(a) the level of one or more free amyloid beta peptides in a biological sample of said subject,

(b) the aggregate levels of a one or more free amyloid peptides in a biological sample of said subject and of said one or more amyloid beta peptides associated to macromolecular components present in said biological sample, wherein said aggregate levels are determined by quantifying the amount of said one or more amyloid beta peptides in cell-free fraction of said sample after contacting said sample with a protein solubilising agent under conditions adequate to promote dissociation of the amyloid beta peptide or peptides from the components present in the biological sample,

(c) the level of one or more amyloid beta peptides associated to cells in a biological sample of said subject, wherein said level is determined by isolating the cell fraction of said biological sample, contacting said cellular fraction of said sample with a protein solubilising agent under conditions adequate to promote dissociation of the amyloid beta peptide or peptides from the cells present in the sample and

wherein the biological sample is plasma or blood, and wherein the parameters are one or more of the parameters selected from the group of

(a) 1ab40, corresponding to the level of free ABETA40 peptide in a biological sample of said subject,

(b) 1ab42, corresponding to the level of free ABETA42 peptide in a biological sample of said subject,

(c) 2ab40, corresponding to the aggregate levels of free ABETA40 peptide in a biological sample of said subject and of ABETA40 peptide associated to components of said biological sample, wherein 2ab40 is determined by quantifying the amount of ABETA40 peptide in said sample after contacting said sample with a protein solubilising agent under conditions adequate to promote dissociation of the ABETA40 peptide from the components present in the biological sample,

(d) 2ab42, corresponding to the aggregate level of free ABETA42 peptide in a biological sample of said subject and of ABETA42 peptide associated to components of said biological sample, wherein 2ab42 is determined by quantifying the amount of ABETA42 peptide in said sample after contacting said sample with a protein solubilising agent under conditions adequate to promote dissociation of the ABETA42 peptide from the components present in the biological sample,

(e) 3ab40, corresponding to the level of the ABETA40 peptide associated to cells in a biological sample of said subject, wherein 3ab40 is determined by quantifying the amount of ABETA40 peptide after contacting the cellular fraction of said biological sample with a protein solubilising agent under conditions adequate to promote dissociation of the amyloid beta peptides from the cells present in the sample and

(f) 3ab42, corresponding to the level of the ABETA42 peptide associated to cells in a biological sample of said subject, wherein 3ab42 is determined by quantifying the amount of ABETA42 peptide after contacting the cellular fraction of said biological sample with a protein solubilising agent under conditions adequate to promote dissociation of the amyloid beta peptides from the cells present in the sample.

The term "amyloid beta peptide" is used herein interchangeably with "Abeta", "Abeta," "beta AP," "A beta peptide," or "Aβ peptide" and refers to a family of peptides that are the principal chemical constituent of the senile plaques and vascular amyloid deposits (amyloid angiopathy) found in the brain in patients of Alzheimer's disease (AD), Down's Syndrome, and Hereditary Cerebral Hemorrhage with Amyloidosis of the Dutch-Type (HCHWA-D). Amyloid beta peptides are fragments of beta-amyloid precursor protein (APP) which comprises a variable number of amino acids, typically 38-43 amino acids.

Amyloid beta peptides are commonly expressed as "Aβ (x-y)" wherein x represents the amino acid number of the amino terminus of the amyloid beta peptide and y represents the amino acid number of the carboxy terminus. For example, Aβ(1-40) is an amyloid beta peptide whose amino terminus begin at amino acid number 1 and carboxy terminus ends at amino acid number 40, a sequence of which is given by SEQ ID NO: 1. The amyloid beta peptides which are detected according to the method of the invention are Aβ(1-40) (SEQ ID NO:1) and Aβ(1-42) (SEQ ID NO;5).

The term "Aβ(1-42)", as used herein, relates to a 42 amino acids peptide corresponding to amino acids 672 to 713 (SEQ ID NO:5) of APP and which is produced by the sequential proteolytic cleavage of the amyloid precursor protein (SEQ ID NO:15) by the β- and γ-secretases.

The term "Aβ(1-40)", as used herein, relates to a 40 amino acids peptide corresponding to amino acids 672 to 711 (SEQ ID NO:1) and which is produced by the sequential proteolytic cleavage of the amyloid precursor protein (SEQ ID NO:15) by the β- and γ-secretases.

The term "biological sample", as understood in the present invention, includes (1) biological fluids such as whole blood, serum and plasma; and (2) blood components, such as plasma, serum, blood cells, and platelets; Methods of the invention are particularly suitable for measuring Aβ in a sample of blood of a human or non-human animal, such as whole blood, plasma, or a sample containing any blood components in any amounts.

The term "whole blood" means blood from a human or animal containing both cellular components and liquid component. Whole blood can be in coagulated state or non-coagulated state. "Whole blood" also includes blood wherein portion or all of the cellular components, such as white blood cells or red blood cells, have been lysed.

The term "plasma" refers to the fluid component of the whole blood. Depending on the separation method used, plasma may be completely free of cellular components, or may contain various amounts of platelets and/or small amount of other cellular components.

The term "serum" refers to plasma without the clotting protein fibrinogen and other clotting factors.

The term "free amyloid beta peptide", as used herein, refers to the amyloid beta peptides which are not associated to any component of the biological sample and which is readily available for binding to a specific antibody. This peptide may be determined by conventional immunological techniques by contacting the biological sample with an antibody specific for said peptide. In a preferred embodiment, the level of free amyloid peptide is determined in plasma.

The term "amyloid beta peptide associated to macromolecular components", as used herein, refers to the amyloid beta peptide which is non-covalently bound or attached to molecules found in the biological sample under study. This peptide is usually not readily accessible for immunological detection and thus, requires a pretreatment of the biological sample in order to achieve the separation of the peptide from the components. Under these conditions, the amyloid beta peptide attached to macromolecular components will be released from said components and will become available for immunological detection using specific antibodies. Since the biological sample contains already a certain amount of free amyloid beta peptide, the total amount of free amyloid peptide after contacting the sample with the protein solubilising agent will be the aggregate level of free amyloid beta peptide originally present and the level of amyloid beta peptide which has been released upon treatment with the protein solubilising agent. In case the level of amyloid beta peptide associated to macromolecular components present in the biological sample needs to be determined, this can be typically done by determining the level of free amyloid beta peptide prior to the treatment with the protein solubilising agent and the level of free amyloid beta peptide after the treatment with the protein solubilising agent and substract the first value from the second value. For the purposes of the present invention, it is usually adequate to determine the aggregated level of free amyloid beta peptides which includes the originally free amyloid beta peptides as well as the level of amyloid beta peptides which have been released from the macromolecular components after the treatment with the protein solubilising agent. Therefore, the parameter which is usually determined when the sample is treated so as to dissociate the amyloid peptide from macromolecular components corresponds to the addition of the free peptide present in the sample and the peptide associated to macromolecular components.

The macromolecular components of the sample which may bind amyloid beta peptides and which contribute to the pool of amyloid beta peptide associated to macromolecular components includes both proteins as well as lipids. In the particular case that the method is carried out in blood or plasma samples, the macromolecular components include, without limitation, blood proteins and lipids. Exemplary blood proteins include albumin, immunoglobulin G, immunoglobulin E, immunoglobulin M, immunoglobulin A, fibrinogen (fibrin and degradation products thereof), alpha-1 antitrypsin, prealbumin, alpha 1 antitrypsin, alpha 1 acid glycoprotein, alpha 1 fetoprotein, Haptoglobin alpha 2, macroglobulin, ceruloplasmin, transferrin, C3/C4 Beta 2 microglobulin, beta lipoprotein, alpha, beta and gamma globulins, C-reactive protein (CRP), prothrombin, thyroxine-binding protein, transthyretin and the like. Exemplary blood lipids include free fatty acids, cholesterol, triglycerides, phospholipids, sphingolipids and the like. The amount of amyloid beta peptide associated to macromolecular components can be determined by contacting a cell-free sample of the biological sample with a protein solubilising agent under conditions adequate for inducing the release of said amyloid beta peptides from the macromolecular components.

By contacting, it is meant herein adding to the sample a sufficient amount of a solution comprising the protein solubilising agent so that the concentration of the protein solubilising agent in the mixture is sufficient to effectively solubilise the amyloid beta peptide which is bound to the proteins and cells in the sample. Preferably, the protein solubilising agent is found in solution in a buffer solution so that the addition of the protein solubilising agent does not result in a substantial modification in the pH of the sample.

The term "protein solubilising agent", as used herein, refers to any compound of composition capable of altering the secondary, tertiary and/or quaternary structure of polypeptides while leaving the primary structure intact. By virtue of these properties, protein solubilising agents are capable increasing the solubility of proteins in a sample as well as of preventing inter- and intramolecular aggregation of proteins. Protein solubilising agents suitable for use in the present invention include, without limitation, detergents, chaotropic agents, reducing agents or mixtures thereof.

The term "detergent", as used herein, is a synonym used for surfactants in general, and refers to amphipathic surface-active agents that, when added to a liquid, reduce surface tension of the liquid in comparison to the same liquid in the absence of the detergent. Detergents are also capable of preventing aggregation of proteins and of preventing nonspecific interaction or binding of contaminants to a protein of interest. Detergents suitable for use in the present invention include, without limitation, non-ionic (neutral), anionic, cationic, or zwitterionic detergents.

Examples of non-ionic or neutral detergents include, without limitation, detergents of the Tween series, such as Tween® 20, Tween® 21, Tween® 40, Tween® 60, Tween® 61, Tween® 65, Tween® 80, Tween® 81, Tween® 85, detergents of the Span® series, such as Span® 20; detergents of the Tergitol series, such as Tergitol Type 15-S-12; detergents of the Brij® series, such as Brij® 35, Brij® 56, Brij® 72, Brij® 76, Brij® 92V, Brij® 97, Brij® 58P; detergents of the Triton® series, such as Triton® X-100, Triton® X-114, Triton® CF-21, Triton® CF-32, Triton® DF-12, Triton® DF-16, Triton® GR-5M, Triton® X-102, Triton® X-15, Triton® X-151, Triton® X-207, Triton® X-165, Triton® X-305, Triton® X-405, Triton® X-45, Triton® X-705-70, or a non-ionic conservative variant of at least one of said detergent.

Examples of anionic detergents include, without limitation, cholic acid and derivatives thereof, taurocholic acid, Triton X-200, Triton W-30, Triton- 30, Triton-770, dioctyl sulfo succinate, N$_5$N- dimethyldodecylamine N-oxide, sodium 1-alkylsulfonates, N-lauroylsarcosine or fatty acid salts.

Examples of cationic detergents includes, without limitation, mono and di-methyl fatty amines, alkyl trimethyl ammonium salts, dialkyl dimethyl ammonium salts, alkyl amine acetates, trialkylammonium acetates, alkyldimethylbenzyl ammonium salts, dialkymethylbenzyl ammonium salts, alkylpyridinium halide and alkyl (alkyl substituted) pyridinium salts, alkylthiomethylpyridinium salts, alkylamidomethylpyridinium salts, alkylquinolinium salts, alkylisoquinolinium salts, N,N-alkylmethylpyrollidonium salts, 1,1-dialkylpiperidinium salts, 4,4-dialkylthiamorpholinium salts, 4,4-dialkylthiamorpholinium-1-oxide salts, methyl his (alkyl ethyl)-2-alkyl imidazolinium methyl sulfate (and other salts), methyl bis(alkylamido ethyl)-2-hydroxyethyl ammonium methyl sulfate (and other salts), alkylamidopropyl-dimethylbenzyl ammonium salts, carboxyalkyl-alkyldimethyl ammonium salts, alkylamine oxides, alkyldimethyl amine oxides, poly(vinylmethylpyridinium) salts, poly(vinylpyridine) salts, polyethyleneimines, trialkyl phosphonium bicarbonates (and other salts), trialkylmethyl phosphonium salts, alkylethylmethylsulfonium salts, and alkyldimethylsulfoxonium salts.

Examples of zwitterionic detergents include, without limitation, 3-[(3-cholamidopropyl)dimethylammonio]-1-propanesulfonate (CHAPS); 3-[(3-cholamidopropyl)dimethylammonio]-2-hydroxy-1-rhoropanesulfonate (CHAPSO); N-(alkyl C10-C16)-N,N-dimethylglycine betaine (EMPIGEN BB); Caprylyl sulfobetaine (SB3-10); 3-[N,N-dimethyl(3-myristoylaminopropyl)ammonio]propanesulfonate (Amidosulfobetaine-14; ASB-14); N-tetradecyl-N,N-dimethyl-3-ammonio-1-propoanesulfonate(3-14 Detergent; ZWITTERGENT); N-dodecyl-N,N'-dimethyl-3-ammonio- 1 -propanesulfonate; N-octadecyl-N,N-dimethyl-3 -ammonio- 1-propanesulfonate; N-decyl-N,N-dimethyl-3 -ammonium- 1 -propanesulfonate; Mirataine CB; Mirataine BB; Mirataine CBR; Mirataine ACS; Miracare 2MHT and Miracare 2MCA.

In a preferred embodiment, the protein solubilising reagent is a detergent. In a still more preferred embodiment, the detergent is Tween 20. In a still more preferred embodiment, Tween 20 is used at a concentration of 0.5%.

A "chaotropic agent", as used herein, relate to a compound or mixture of compounds which disrupt hydrogen bonds and hydrophobic interactions both between and within proteins. When used at high concentrations, chaotropic agents disrupt secondary protein structure and bring into solution proteins that are not otherwise soluble. Suitable chaotropic agents include, without limitation, urea, guanidinium isothiocyanate, sodium thiocyanate (NaSCN), Guanidine HCl, guanidinium chloride, guanidinium thiocyanate, lithium tetrachloroacetate, sodium perchlorate, rubidium tetrachloroacetate, potassium iodide or cesium trifluoroacetate.

The term "reducing agent", as used herein, refers to any compound or material which maintains sulfhydryl groups in the reduced state and reduces intra- or intermolecular disulfide bonds. By way of example, reducing agents suitable for the method of the present invention include both sulfhydryl or phosphine reducing agents. Examples of sulfhydryl reductants include dithiothreitol (DTT), dithioerythritol (DTE), and β-mercaptoethanol. Examples of phosphine reductants include tributylphosphine (TBP) and tris-carboxyethylphosphine (TCEP).

Typically, the biological sample is first processed to remove the cellular fraction. The cell-free sample is then contacted with the protein solubilising agent. In a preferred embodiment, the sample is diluted using a buffer comprising the protein solubilising agent. Typically, the sample is diluted 5-fold in a buffer solution comprising Tween 20.

As used herein, a "buffer solution" is any substance or mixture of compounds in solution that is capable of neutralizing both acids and bases without appreciably changing the original acidity or alkalinity of the solution. Suitable buffer solutions to be used in the method of the invention include, without limitation, Tris buffer solution, phosphate buffer solution, borate buffer solution, carbonate buffer solution, glycine-sodium hydroxide buffer solution, or the like. Preferably, the buffer solution is a phosphate buffer solution such as phosphate-buffered saline or PBS.

The amount of solution comprising the protein solubilising agent which is added to the biological sample is not essential as long as sufficient dissociation of the amyloid beta peptide is achieved. By way of example, the biological fluid may be diluted in the solution comprising the protein solubilising agent at a dilution of at least 1/2 (v/v), 1/3 (v/v), 1/4 (v/v), 1/5 (v/v), 1/6 (v/v), 1/7 (v/v), 1/8 (v/v), 1/9 (v/v), 1/10 (v/v), 1/20 (v/v), 1/50 (v/v), 1/60 (v/v), 1/80 (v/v), 1/90 (v/v), 1/100 (v/v) or more. The skilled person will appreciate that any combination of said dilution rates and of said protein solubilising agent concentration can be used as long as the final concentration of protein solubilising agent is adequate for achieving the desired effect. For instance, the solution containing the protein solubilising agent may comprise said selected protein solubilising agent (s) at a concentration ranging from 0.001% to 0.5% (w/v). After having been diluted in said solution containing the protein solubilising agent, said biological fluid typically contains said surfactant(s) at less than 0.1% (w/v), preferably less than 0.6% (w/v), more preferably no more than 0.5% (w/v), most preferably no more than 0.45% (w/v) and even most preferably 0.5%.

Suitable buffer systems for use in the present invention include Tris-HCl buffers including a salt such as NaCl or KCl and, optionally, BSA. Particular buffer systems include, without limitation,

50 mM Tris-HCl pH 8, 0.5M NaCl, 0.05 % BSA, 0.05 % Tween-20;
50 mM Tris-HCl pH 8, 0.5M NaCl, 0.05 % BSA, 0.05 % Tween-20, 1M GuHCl;
50 mM Tris-HCl pH 8, 0.5M KCl, 0.05 % BSA, 0.05 % Tween-20 ;
50 mM Tris-HCl pH 8, 0.5M KCl, 0.05 % BSA, 0.05 % Tween-20, 1M GuHCl;
50 mM Tris-HCl pH 8, 0.5M NaCl, 0.05 % BSA, 0.05 % Tween-80;
50 mM Tris-HCl pH 8, 0.5M KCl, 0.05 % BSA, 0.05 % Tween-80;
50 mM Tris-HCl pH 8, 0.5M NaCl; 0.05 % BSA, 0.05 % Triton X-100;
50 mM Tris-HCl pH 8, 0.5M KCl, 0.05 % BSA, 0.05 % Triton X-100;
50 mM Tris-HCl pH 8; 0.05 % BSA, 0.05 % Tween-20;
50 mM Tris-HCl pH 8, 0.5M NaCl, 0.1 % BSA, 0.05 % Tween-20;
50 mM Tris-HCl pH 8, 0.5M NaCl, 0.05 % BSA, 0.1 % Tween-20;
50 mM Tris-HCl pH 8, 0.5M NaCl, 0.1 % BSA, 0.1 % Tween-20;
50 mM Tris-HCl pH 8, 1M NaCl, 0.05 % BSA, 0.05 % Tween-20;
50 mM Tris-HCl pH 8, 1.5M NaCl, 0.05 % BSA, 0.05 % Tween-20;
50 mM Tris-HCl pH 8, 2M NaCl, 0.05 % BSA, 0.05 % Tween-20;
50 mM Tris-HCl pH 8, 2.5M NaCl, 0.05 % BSA, 0.05 % Tween-20;
50 mM Tris-HCl pH 8, 3M NaCl, 0.05 % BSA, 0.05 % Tween-20;
50 mM Tris-HCl pH 8, 0.5M NaCl, 0.05 % BSA, 0.05 % Tween-20, 10% DMSO;
50 mM Tris-HCl pH 8, 0.5M NaCl, 0.05 % BSA, 0.05 % Tween-20, 0.5 M GuHCl;
50 mM Tris-HCl pH 6, 0.5M NaCl, 0.05 % BSA, 0.05 % Tween-20;
50 mM Tris-HCl pH 7, 0.5M NaCl, 0.05 % BSA, 0.05 % Tween-20;
50 mM Tris-HCl pH 9, 0.5M NaCl, 0.05 % BSA, 0.05 % Tween-20;
50 mM Tris-HCl pH 8, 0.5M NaCl, 0.05 % BSA

For example, when Tween 20 is used as a protein solubilisng agent, the preferred concentration is of 0.004-0.02%, more preferably of 0.005-0.01 % (w/v).

The contacting step is carried out preferably at a low temperature in order to inhibit proteolytic activities present in the sample. Suitable temperatures are of about 0-10 Degrees C, preferably of about 3-5 Degrees C, e.g., about 4 Degrees C.

Once the biological fluid has been contacted with the solution comprising the protein solubilising agent, both fluids may be mixed. Mixing may be carried out by stirring, preferably by shaking, more preferably by high speed shaking, most preferably by vortexing) for at least 5 seconds, preferably for at least 10 seconds, more preferably for at least 15 seconds (e.g., for 15-50 seconds). Advantageous speeds for said mixing, stirring, shaking, high speed shaking or vortexing comprise a speed of at least 250 rpm, preferably of at least 500 rpm, more preferably of at least 1,000 rpm, most preferably of about 2,000-2,500 rpm.

The contacting step is carried out under conditions adequate for achieving partial or, preferably, full dissociation of the amyloid beta peptide from the protein and lipids present in the biological sample. The conditions can be adequately determined by one of ordinary skills in the art by monitoring the amount of amyloid beta peptide which is detectable before the contacting step and progressively at different time points after the contacting step has taken place. The time course experiment may be determined as described in example of the experimental part.

The skilled person will appreciate that when the level of amyloid beta peptide is determined by diluting a biological sample with a buffer containing the protein solubilising reagent, the level of free amyloid beta peptide obtained by immunological determination will have to be corrected in order to take into consideration the dilution factor previously applied to the biological sample.

Thus, in a preferred embodiment, the parameter which is determined in step (i) of the method of the invention is one or more of the parameter selected from the group of the level of free ABETA40 peptide in a biological sample of said subject (hereinafter known as 1ab40 or UP A$\beta$(1-40)), the aggregate levels of free ABETA40 peptide in the biological sample and of ABETA40 peptide associated to components of said biological sample obtained as described above (hereinafter referred to as 2ab40 or DP A$\beta$(1-40)), the level of free ABETA42 peptide in the biological sample (hereinafter known as 1ab42 or UP A$\beta$(1-42)) and the aggregate levels of free ABETA42 peptide in the biological sample and of ABETA42 peptide associated to components of said biological sample obtained as described above (hereinafter referred to as 2ab42 or DP A$\beta$(1-42)).

The term "amyloid beta peptide associated to cells", as used herein, refers to amyloid beta peptide which is non-covalently associated to the surface of the cells present in the biological sample and which is unavailable for binding to antibodies added to the sample and hence, immunologically undetectable. Typically, if the biological sample is blood, the amyloid beta peptide is associated to red blood cells, white blood cells, including neutrophils, eosinophils, basophils, lymphocytes and monocytes, and platelets.

The amount of amyloid beta peptide associated to cells in a given sample can be determined and this value can be used alone or in combination with other parameters related to amylioid beta peptides in the methods of the invention. For this purpose, it is first required to isolate the cellular fraction from the biological sample. This can be carried out using any technique known to the skilled person such as centrifugation, sedimentation, filtration and the like. Once the cell fraction of a biological sample has been isolated, the cells are contacted with a protein solubilising agent.

Suitable protein solubilising agent include detergents, chaotropic agents and reducing agents as defined above and are usually provided in a buffer solution at an adequate concentration. Suitable agents, buffer solutions and concentrations of agents in the buffer solution have been described above. The contacting step is carried out essentially as explained above in the method for releasing the amyloid peptide which is attached to components (proteins and lipids) of the biological sample. In a preferred embodiment, the protein solubilising agent is a detergent. In a still more preferred embodiment, the detergent is Tween 20. Suitable concentrations of Tween 20 for use as protein solubilising agent are as defined above, i.e. between 0.004-0.02%, more preferably of 0.005-0.01 % (w/v).

The contacting step is carried out preferably at a low temperature in order to inhibit proteolytic activities present in the sample. Suitable temperatures are of about 0-10 Degrees C, preferably of about 3-5 Degrees C, e.g., about 4 Degrees C.

Typically, the contacting step is carried out by resuspending the cellular fraction in the biological sample with the solution comprising the protein solubilising agent. Said resuspension can be carried out by gentle pippeting up- and down, by stirring, preferably by shaking, more preferably by high speed shaking, most preferably by vortexing for at least 5 seconds, preferably for at least 10 seconds, more preferably for at least 15 seconds (e.g., for 15-50 seconds). Advantageous speeds for said mixing, stirring, shaking, high speed shaking or vortexing comprise a speed of at least 250 rpm, preferably of at least 500 rpm, more preferably of at least 1,000 rpm, most preferably of about 2,000-2,500 rpm.

The contacting step is carried out under conditions adequate for achieving partial or, preferably, full dissociation of the amyloid beta peptide from the cells present in the biological sample. The conditions can be adequately determined by one of ordinary skills in the art by monitoring the amount of amyloid beta peptide which is detectable before the contacting step and progressively at different time points after the contacting step has taken place. The time course experiment may be determined as described in example of the experimental part.

Thus, in a preferred embodiment, the parameter which is determined in step (i) of the method of the invention is one or more of the parameter selected from the group of the level of ABETA40 associated to cells present in the biological sample (hereinafter known as 3ab40 or CB $A\beta$(1-40)) and the level of ABETA42 associated to cells present in the biological sample (hereinafter known as 3ab42 or CB $A\beta$(1-42)).

The following step of the method of the invention comprises comparing the value of at least one of the parameters (b) or (c) or the value of a calculated parameter resulting from arithmetically combining one or more of the parameters (a) to (c) with a reference value corresponding to the value of said parameters (b) or (c) or said calculated parameter in a reference sample wherein (a), (b) and (c) have been defined in detail above and correspond, respectively, to the level of a free amyloid beta peptide in a biological sample of said subject (a), the aggregate levels of a free amyloid peptide in a biological sample of said subject and of said amyloid beta peptide associated to macromolecular components present in said biological sample (b) and the level of an amyloid beta peptide associated to cells in a biological sample of said subject (c).

The parameters which are used in step (ii) of the method of the invention are either direct parameters, i.e. parameters which can be determined directly as defined above and which correspond to those previously defined as 2ab40, 3ab40, 2ab42 and 3ab42. Alternatively, it is also possible to combine arithmetically one or more of the direct parameters in order to obtain a calculated parameter. In practice, any arithmetic combination of two more parameters may yield calculated parameters with diagnostic value including additions, substractions, multiplications, divisions and combinations thereof. Particular calculated markers for use in the method of the present invention include, without limitation, 2ab40/2ab42, 3ab40/3ab42, 2ab40/3ab40, 2ab42/3ab42, 1ab40 + 2ab40, 1ab40 + 3ab40, 2ab40 + 3ab40, 1ab40 + 2ab40 + 3ab40, 1ab42 + 2ab42, 1ab42 + 3ab42, 2ab42 + 3ab42, 1ab42 + 2ab42 + 3ab42, 1ab40 + 2ab40 + 1ab42 + 2ab42, 1ab40 + 3ab40 + 1ab42 + 3ab42, 2ab40 + 3ab40 + 2ab42 + 3ab42, 1ab40 + 2ab40 + 3ab40 + 1ab42 + 2ab42 + 3ab42, (1ab40 + 2ab40)/(1ab42 + 2ab42), (1ab40 + 3ab40)/(1ab42 + 3ab42), (2ab40 + 3ab40)/(2ab42 + 3ab42), (1ab40 + 2ab40 + 3ab40)/(1ab42 + 2ab42 + 3ab42), (1ab42 + 2ab42)/(1ab40 + 2ab40), (1ab42 + 3ab42)/(1ab40 + 3ab40), (2ab42 + 3ab42)/(2ab40 + 3ab40), (1ab42 + 2ab42 + 3ab42)/(1ab40 + 2ab40 + 3ab40), 2ab40 -1ab40, 2ab42-1ab42 y (2ab40-1ab40)/(2ab42-1ab42). In a preferred embodiment, the calculated parameter results from the addition of the 2ab40 and 3ab40 parameters (hereinafter referred to as T40). In another preferred embodiment, the calculated parameter results from the addition of the 2ab42 and 3ab42 parameters (hereinafter referred to as T42). In yet another preferred embodiment, the calculated parameter results from the addition of the 2ab40, 3ab40, 2ab42 and 3ab42 parameters (hereinafter referred to as T-$\beta$APB).

The term "reference value", as used herein, refers to a value of the parameter which is being used for comparison and which has been determined in a subject not suffering from a neurodegenerative disease or without any history of neurodegenerative disease. Preferably, the subjects from which the reference values for the different parameters and calculated parameters are obtained are patients which show an absence of memory complains, normal performance in neuropsychological tests and absence of structural alterations in MRI.

In particular, reference values are selected which allow a sensitivity higher than 85% and a specificity higher than 75%. In another preferred embodiment, the reference values are selected so as to obtain a sensitivity higher than 70% and an specificity higher than 70%. Preferably, the reference values allow obtaining a prediction with an accuracy or precision of at least 80%.

In step (iii) of the method of the invention, the diagnosis of the neurodegenerative disease, the detection of a stage prior to a neurodegenerative disease or the distinction of a neurodegenerative disease from a stage prior to a neurodegenerative disease is carried out using a particular set of markers or calculated markers which provide particularly adequate specificity and sensitivity levels. Thus, in a particular example, the diagnosis of a neurodegenerative disease is carried out by comparing the value of a parameter selected from the group of 3ab40 and 2ab42 or the value of a calculated parameter selected from the group of 2ab40 + 3ab40 and 2ab40 + 3ab40 + 2ab42 + 3ab42.

In another particular embodiment, the detection of a stage prior to a neurodegenerative disease is carried out by comparing the value of a parameter selected from the group of 2ab40, 3ab40 and 2ab42 or the value of a calculated parameter selected from the group of 2ab40 + 3ab40, 2ab40 + 3ab40 + 2ab42 + 3ab42, 1ab40 + 2ab40 + 3ab40 + 1ab42 + 2ab42 + 3ab42, 1ab40 + 2ab40 + 3ab40, 1ab42 + 2ab42 + 3ab42, 1ab40 + 1ab42 + 2ab42 + 3ab42, 1ab40 + 2ab40 + 1ab42 + 2ab42 and 1ab40 + 3ab40 + 1ab42 + 3ab42.

In yet another embodiment, the distinguishing of a neurodegenerative disease from a stage prior to said neurodegenerative disease is carried out by comparing the value of a parameter selected from the group of 3ab40 and 2ab42 or by comparing the value of a calculated parameter selected from the group of 2ab40 + 3ab40, 2ab40 + 3ab40 + 2ab42 + 3ab42 and 1ab40 + 2ab40 + 1ab42 + 2ab42.

Adequate reference values for the different diagnostic methods of the invention are summarized in Table 1.

| Method | Parameter | Cut-off value (pg/ml) |
|---|---|---|
| Detection of a stage prior to a neurodegenerative disease | 2ab40 /DP (Aβ1-40) | 63.8 |
| Diagnosis of a neurodegenerative disease | 3ab40/CB (Aβ1-40) | 71.9 |
| Detection of a stage prior to a neurodegenerative disease | 3ab40/CB (Aβ1-40) | 71.1 |
| Distinguishing a neurodegenerative disease from a stage prior to said neurodegenerative disease | 3ab40/CB (Aβ1-40) | 211.3 |
| Diagnosis of a neurodegenerative disease | 2ab42/DP (Aβ1-42) | 47.4 |
| Detection of a stage prior to a neurodegenerative disease | 2ab42/DP (Aβ1-42) | 50.3 |
| Distinguishing a neurodegenerative disease from a stage prior to said neurodegenerative disease | 2ab42/DP (Aβ1-42) | 151.7 |
| Diagnosis of a neurodegenerative disease | 3ab42/CB (Aβ1-42) | 76.9 |
| Detection of a stage prior to a neurodegenerative disease | 3ab42/CB (Aβ1-42) | 58.8 |
| Diagnosis of a neurodegenerative disease | 2ab40 + 3ab40/T40 | 132.7 |
| Detection of a stage prior to a neurodegenerative disease | 2ab40 + 3ab40/T40 | 132.7 |
| Distinguishing a neurodegenerative disease from a stage prior to said neurodegenerative disease | 2ab40 + 3ab40/T40 | 550.8 |
| Diagnosis of a neurodegenerative disease | 2ab42 + 3ab42/T42 | 115.8 |
| Detection of a stage prior to a neurodegenerative disease | 2ab42 + 3ab42/T42 | 103.3 |
| Diagnosis of a neurodegenerative disease | 2ab40 + 3 ab40 + 2ab42 + 3ab42/T-βAPB | 235.5 |
| Detection of a stage prior to a neurodegenerative disease | 2ab40 + 3ab40 + 2ab42 + 3ab42/T-βAPB | 235.5 |
| Distinguishing a neurodegenerative disease from a stage prior to said neurodegenerative disease | 2ab40 + 3ab40 + 2ab42 + 3ab42/T-βAPB | 778.1 |
| Detection of a stage prior to a neurodegenerative disease | 1ab40 + 2ab40 + 3ab40 + 1ab42 + 2ab42 + 3 ab42 | 272.1 |

(continued)

| Method | Parameter | Cut-off value (pg/ml) |
|---|---|---|
| Detection of a stage prior to a neurodegenerative disease | 1ab40 + 2ab40 + 3ab40 | 155.8 |
| Detection of a stage prior to a neurodegenerative disease | 1ab42 + 2ab42 + 3ab42 | 124.3 |
| Diagnosis of a neurodegenerative disease | 1ab40 + 2ab40 + 1ab42 + 2ab42 | 158.3 |
| Detection of a stage prior to a neurodegenerative disease | 1ab40 + 2ab40 + 1ab42 + 2ab42 | 142.4 |
| Distinguishing a neurodegenerative disease from a stage | 1ab40 + 2ab40 + 1ab42 + | 161.2 |

Table 1: Summary of preferred methods of the invention according to the parameter and the cut-off value.

| prior to said neurodegenerative disease | 2ab42 | |
|---|---|---|
| Detection of a stage prior to a neurodegenerative disease | 1ab40 + 3ab40 + 1ab42 + 3ab42 | 154.7 |

Once the value of the parameter or the calculated parameter is determined, the diagnosing of a neurodegenerative disease, the detecting of a stage prior to a neurodegenerative disease or the distinguishing of a neurodegenerative disease from a stage prior to said neurodegenerative disease according to the invention is carried out when there is an alteration in the value of the parameter or in the value of the calculated parameter with respect to the reference value.

The term "alteration" refers to an statistically significant increase or decrease in the value of the parameter under consideration with respect to the reference value.

By "statistically significant", as used herein, relate to a statistical analysis of the probability that there is a non-random association between two or more results, endpoints or outcome, i.e. that there is a certain degree of mathematical assurance that the value of the parameter is associated with a particular patient population with respect to the reference value.

The statistical significance of the alteration in the values can be determined using p-value, For instance, when using p-value, a parameter is identified as showing a significant alteration when the p-value is less than 0.1, preferably less than 0.05, more preferably less than 0.01, even more preferably less than 0.005, the most preferably less than 0.001.

Typically, the value of the parameter under consideration can be assigned as being "increased" when the value above the reference value is of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value. On the other hand, a parameter value can be considered as being "decreased" when it is at least 0.9-fold, 0.75-fold, 0.2-fold, 0.1-fold, 0.05-fold, 0.025-fold, 0.02-fold, 0.01-fold, 0.005-fold or even less compared with reference value. In a particular embodiment, the alteration in the value of the parameter or in the value of the calculated parameter with respect to the reference value is an increase.

Any method suitable for the determination of peptides can be used in the present invention. By way of example, the concentration of amyloid beta peptide can be determined using one or more techniques chosen from Western blot, immunoprecipitation, enzyme-linked immunosorbent assay (ELISA), surface plasmon resonance, precipitin reaction, a gel diffusion immunodiffusion assay, radioimmunoassay (RIA), fluorescent activated cell sorting (FACS), two-dimensional gel electrophoresis, capillary electrophoresis, mass spectroscopy (MS), matrix- assisted laser desorption/ionization-time of flight-MS (MALDI-TOF), surface- enhanced laser desorption ionization-time of flight (SELDI-TOF), high performance liquid chromatography (HPLC), fast protein liquid chromatography (FPLC), multidimensional liquid chromatography (LC) followed by tandem mass spectrometry (MS/MS), thin-layer chromatography, protein chip expression analysis and laser densitometry.

In a preferred embodiment, the determination of the at least one or more of 1ab40, 1ab42, 2ab40, 2ab42, 3ab40 and 3ab42 is carried out by an immunological method. As used herein, "immunological method", when applied to a determination, relates to any method which involves the use of one or more antibodies specific for a target substance in order to determine the amount/concentration of said target substance excluding other substances found in the sample. Suitable immunological methods include, without limitation, Western blot, immunoprecipitation, enzyme-linked immunosorbent assay (ELISA), surface plasmon resonance, radioimmunoassay (RIA).

The skilled person will appreciate that any type of antibody is adequate for performing the immunological detection methods according to the invention provided that the antibody is specific enough to effectively discriminate the amyloid beta peptide species in the sample from other substances. Antibodies molecules suitable for use in the immunological methods of the invention include, without limitation:

(i) "intact" antibodies which comprise an antigen-binding variable region as well as a light chain constant domain (CL) and heavy chain constant domains, CH1, CH2 and CH3,

(ii) "Fab" fragments resulting from the papain digestion of an intact antibody and which comprise a single antigen-binding site and a CL and a CH1 region,

(iii) "F(ab')$_2$" fragments resulting from pepsin digestion of an intact antibody and which contain two antigen-binding sites,

(iv) "Fab'" fragments contain the constant domain of the light chain and the first constant domain (CH1) of the heavy chain and has one antigen-binding site only. Fab' fragments differ from Fab fragments by the addition of a few residues at the carboxy terminus of the heavy chain CH 1 domain including one or more cysteines from the antibody hinge region.

(v) "Fv" is the minimum antibody fragment which contains a complete antigen-recognition and antigen-binding site. This region consists of a dimer of one heavy chain and one light chain variable domain in tight, non-covalent-association. It is in this configuration that the three hypervariable regions (CDRs) of each variable domain interact to define an antigen-binding site on the surface of the VH - VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

(vi) Single-chain FV or "scFv" antibody fragments comprise the VL and VH, domains of antibody, wherein these domains are present in a single polypeptide chain. Preferably, the VL and VH regions are connected by a polypeptide linker which enables the scFv to form the desired structure for antigen binding.

(vii) "Diabodies" comprise a heavy-chain variable domain (VH) connected to a light chain variable domain (VL) on the same polypeptide chain (VH-VL) connected by a peptide linker that is too short to allow pairing between the two domains on the same chain. This forces pairing with the complementary domains of another chain and promotes the assembly of a dimeric molecule with two functional antigen binding sites.

(viii) "Bispecific antibodies" (BAbs) are single, divalent antibodies (or immunotherapeutically effective fragments thereof) which have two differently specific antigen binding sites. The two antigen sites may be coupled together chemically or by genetic engineering methods known in the art.

All these antibody fragments can be further modified using conventional techniques known in the art, for example, by using amino acid deletion(s), insertion(s), substitution(s), addition(s), and/or recombination (and/or any other modification(s) (e.g. posttranslational and chemical modifications, such as glycosylation and phosphorylation) known in the art either alone or in combination. Methods for introducing such modifications in the DNA sequence underlying the amino acid sequence of an immunoglobulin chain are well known to the person skilled in the art; see, e.g., Sambrook et al.; Molecular Cloning: A Laboratory Manual; Cold Spring Harbor Laboratory Press, 2nd edition 1989 and 3rd edition 2001.

Antibodies comprise both polyclonal and monoclonal antibodies. For the production of polyclonal antibodies, various hosts including goats, rabbits, rats, mice, camels, dromedaries, llamas, humans, birds and others may be immunized by injection with a peptide corresponding to a fragment of Aβ40 or Aβ42 which has immunogenic properties. Depending on the host species, various adjuvants may be used to increase immunological response. Such adjuvants include, but are not limited to, Freund's, mineral gels such as aluminium hydroxide, and surface active substances such as lysolecithin, polyanions, peptides, oil emulsions, KLH, and dinitrophenol. Among adjuvants used in humans, BCG (bacilli Calmette-Guerin) and *Corynebacterium parvum* are especially preferable. If the antigen is a peptide, it may be useful to conjugate it to a protein that is immunogenic in the species to be immunized. For example, the antigen can be conjugated to keyhole limpet hemocyanin (KLH), Blue Carrier (hemocyanin isolated from *Concholepas concholepas*), bovine thyroglobulin, or soybean trypsin inhibitor, using a bifunctional or derivatizing agent, e.g., maleimidobenzoyl sulfosuccinimide ester (conjugation through cysteine residues), N-hydroxysuccinimide (through lysine residues), glutaraldehyde, succinic anhydride or SOCl$_2$.

For the production of monoclonal antibodies, conventional techniques can be used. For instance, monoclonal antibodies may be made using the hybridoma method first described by Kohler et al., Nature, 256:495 (1975) using the procedure described in detail in units 11.4 to 11.11 of Ausubel, F.M. et al. (Current Protocols in Molecular Biology, John Wiley & Sons Inc; ring-bound edition, 2003). Alternatively, monoclonal antibodies can be isolated by recombinant DNA procedures from antibody phage libraries generated using the techniques described in McCafferty et al., Nature, 348:552-554 (1990). Clacksoii et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nucl. Acids. Res., 21:2265-2266 (1993)). Thus, these techniques are viable alternatives to traditional monoclonal antibody hybridoma techniques for isolation of monoclonal antibodies.

Polyclonal antibodies can be used directly as an antiserum obtained from immunised hosts after bleeding and removal of the fibrin clot. Monoclonal antibodies can be used directly as the supernatant of the hybridoma culture or as ascites fluid after implantation of the hybridoma in the peritoneal cavity of a suitable host. Alternatively, the immunoglobulin molecules, either polyclonal or monoclonal, can be purified prior to their use by conventional means such as affinity purification using peptides derived from amyloid beta peptides, non-denaturing gel purification, HPLC or RP-HPLC, size exclusion, purification on protein A column, or any combination of these techniques.

Suitable antibodies for carrying out the immunological methods of the invention include, without limitation:

(i) Antibodies which recognise a region from the N-terminal region of amyloid beta peptides, such as antibodies specific for the epitopes located within amino acids 1 to 16, 1 to 17, 13 to 28, 15 to 24, 1 to 5 and 1 to 11 of A$\beta$40 or A$\beta$42. Antibodies prepared against a peptide corresponding to the C-terminal region of the A$\beta$42 peptide which binds specifically to A$\beta$42 without giving any substantial cross-reaction with A$\beta$40 or A$\beta$43,

(ii) Antibodies prepared against a peptide corresponding to the C-terminal region of the A$\beta$40 peptide which binds specifically to A$\beta$40 without giving any substantial cross-reaction with A$\beta$42, A$\beta$39, A$\beta$38, A$\beta$41 or A$\beta$43,

(iii) Antibodies that recognises simultaneously the C-terminal region of both A$\beta$40 and A$\beta$42 and

(iv) Antibodies specific for the junction region of amyloid beta peptides, which are suitable to distinguish amyloid beta peptides from other APP fragments and which is located within amino acids 16 and 17, tipically spanning amino acids residues 13 to 28.

(v) a combination of two or more of the antibodies mentioned under (i) to (iv).

In a preferred embodiment, the determination of the amount of amyloid beta peptide is carried out by ELISA.

The term "ELISA", as used herein, stands for enzyme-linked immunosorbent assay and relates to an assay by which an unknown amount of target substance (the amyloid beta peptide) is affixed to a surface, and then a specific antibody is washed over the surface so that it can bind to the antigen. This antibody is linked to an enzyme, and in the final step a substance is added that the enzyme can convert to some detectable signal. Different types of ELISA assays are known and can be applied to the method of the invention, including direct ELISA, sandwich ELISA, competitive ELISA and ELISA reverse method & device (ELISA-R m&d).

Direct ELISA is carried out by contacting the test sample comprising the amyloid beta peptide with a solid support which has been previously coated with a concentrated solution of a non-interacting protein or reagent (bovine serum albumin, casein). Once the amyloid beta peptide present in the test sample is absorbed onto the support, an antibody specific for amyloid beta peptide is added under conditions adequate for binding onto the amyloid beta peptide. The antibody which is bound is then detected with a secondary antibody which is coupled to a detectable tag or to a substrate modifying enzyme. The signal resulting from the detectable tag or from the substrate is then proportional to the amount of antibody bound to the support which, in turn, correlates directly with the amount of amyloid beta peptide in the sample.

Competitive ELISA assay includes a first step wherein the test sample comprising an unknown amount of amyloid beta peptide is contacted with a first antibody as defined above. The antibody-antigen complexes are added to an antigen coated well. Once the support is washed to remove any non-specifically bound complexes, the amount of first antibody is detected with a second antibody which is coupled to a detectable moiety. In this type of assays, the higher the original antigen concentration, the weaker the eventual signal. An alternative competitive ELISA assay is that which includes an enzyme-linked antigen rather than enzyme-linked antibody. The labeled antigen competes for primary antibody binding sites with the sample antigen (unlabeled). Using this type of assays, the concentration of antigen in the sample inversely correlates with the amount of labeled antigen retained in the well and, accordingly, in a weaker signal.

ELISA reverse method & device (ELISA-R m&d) uses an innovative solid phase constituted of an immunosorbent polystyrene rod with 4-12 protruding ogives; the entire device is suitable to be introduced in a test tube containing the collected sample and the following steps (washing, incubation in conjugate and incubation in chromogenous) are easily carried out by immerging the ogives in microwells of standard microplates prefilled with reagents, sealed and stored until their use.

In a preferred embodiment, the ELISA assay is an ELISA sandwich assay. The ELISA sandwich assay involves coating a support with a first antibody specific for amyloid beta peptide, applying the sample containing the amyloid beta peptide which will result in the binding of the amyloid beta peptide to the first antibody and applying a second antibody also specific for amyloid beta peptide, wherein said second antibody is usually coupled to a detectable tag or to a substrate-modifying enzyme. The signal generated by the tag or by the converted substrate is the proportional to the amount of antigen in the sample.

In the context of the present invention, the first antibody will be referred to as "capture antibody", meaning that this antibody is used to retrieve from a sample all molecular species to which the antibody specifically binds. There is practically no limitation with regard to the type of antibody that can be used as capture antibody as long as it contains at least one antigen binding site specific for A$\beta$40 and/or A$\beta$42. Thus, any of the antibodies mentioned above may be used as capture antibody.

In the context of the present invention, the second antibody will be referred to as "detection antibody", since this antibody will be used to detect the amount of antigen which has been retained by the capture antibody. As with the capture antibody, there is practically no limitation with regard to the type of antibody that can be used as detection antibody. However, it will be also understood by the person skilled in the art that the detection antibody (i) must bind to a region of the antigen which is not covered by the capture antibody and (ii) must contain not only the antigen binding site but also either a detectable tag, a substrate-modifying enzyme or an additional region or regions that can be specifically detected by a reagent showing high affinity binding for said antibody, so as to allow detection of the antibody which is bound to the antigen captured by the capture antibody. Preferably, said additional regions which can be specifically bound by said reagent correspond to the constant region of the immunoglobulin molecule.

In a preferred embodiment, the capture antibody is an antibody specific against the N-terminal region of the amyloid beta peptides. In a still more preferred embodiment, said capture antibody is an antibody specific against an epitope located within amino acids 1 to 16 of Abeta40 or Abeta42. A particularly preferred capture antibody is the 6E10 monoclonal antibody as described by Kim, K.S. (Neuroscience Res. Comm. 1988, 2:121-130).

In another preferred embodiment, the detection antibody is an antibody specific against an epitope located in the C-terminal region of the amyloid beta peptide. As explained above, the nature of the detection antibody may be adequately chosen by one of ordinary skills in the art depending on the number of amyloid beta species that are to be detected.

In order to detect or determine specifically $A\beta40$, the capture antibody can be an antibody which recognises the N-terminal region of $A\beta40$ (and also of $A\beta42$, since both peptides have identical N-terminal regions) and the detection antibody can be an antibody which recognises specifically the C-terminal region of $A\beta40$ without giving any cross-reaction with $A\beta42$. Alternatively, $A\beta40$ can be specifically detected using a capture antibody which recognises the C-terminal region of $A\beta40$ without giving any cross-reaction with $A\beta42$ and a detection antibody which recognises a region of $A\beta40$ which is common to both $A\beta40$ and $A\beta42$, preferably the N-terminal region of $A\beta42/A\beta40$.

In order to detect or determine specifically $A\beta42$, the capture antibody can be an antibody which recognises the N-terminal region of $A\beta42$ (and also of $A\beta40$, since both peptides have identical N-terminal regions) and the detection antibody can be an antibody which recognises specifically the C-terminal region of $A\beta42$ without giving any cross-reaction with $A\beta40$. Alternatively, $A\beta42$ can be specifically detected using a capture antibody which recognises the C-terminal region of $A\beta42$ and a detection antibody which recognises a region of $A\beta42$ which is common to both $A\beta42$ and $A\beta40$.

In order to detect or determine simultaneously $A\beta42$ and $A\beta40$, the capture antibody can be an antibody which recognises the N-terminal region common to $A\beta42$ and $A\beta40$ and the detection antibody can be a combination of at least two antibodies, wherein the first antibody recognises specifically the C-terminal region of $A\beta42$ without giving any cross-reaction with $A\beta40$ and the second antibody recognises specifically the C-terminal region of $A\beta40$ without giving any cross-reaction with $A\beta42$. Alternatively, capture antibody can be an antibody which recognises the N-terminal region common to $A\beta42$ and $A\beta40$ and the detection antibody can be an antibody that recognises the C-terminal region of both $A\beta40$ and $A\beta42$. Alternatively, $A\beta42$ and $A\beta40$ can be simultaneously detected using as capture antibody a mixture of at least two antibodies comprising a first antibody which recognises specifically the C-terminal region of $A\beta42$ without giving any cross-reaction with $A\beta40$ and a second antibody which recognises specifically the C-terminal region of $A\beta40$ without giving any cross-reaction with $A\beta42$ and a detection antibody which recognises the N-terminal region common to both $A\beta42$ and $A\beta40$. Alternatively, $A\beta42$ and $A\beta40$ can be simultaneously detected using as capture antibody an antibody which recognises the C-terminal region of both $A\beta40$ and $A\beta42$ and a detection antibody which recognises the N-terminal region common to both $A\beta42$ and $A\beta40$.

Antibodies specific for $A\beta40$ and $A\beta42$ and methods for their preparation have been described in detail in WO2004024770 and WO2004098631.

The detection and/or the capture antibodies may have been affinity-purified using a polypeptide which comprises the sequence of the polypeptide used for their preparation.

As mentioned above, the detection antibody may be directly coupled to a detectable tag or to an substrate-modifying enzyme. Preferably, a reagent which shows affinity for the detection antibody may be used, in which case, it is said reagent which is labelled with a detectable tag or with a substrate-modifying enzyme instead of the detection antibody. Moreover, the antibody-binding reagent may be coupled to a first member of a binding pair, in which case, it is the second member of a binding pair which can be coupled to a detectable tag or to a substrate-modifying enzyme.

The antibody-binding reagent may non-covalently bind to a particular type(s), a particular class(es) and/or a particular subclass(es) of an antibody or antibody fragments. Alternatively, the antibody-binding reagent may non-covalently bind to an antibody specific for a particular antigen. In certain embodiments, the antibody-binding reagent binds non-covalently to the Fc region or to the F(ab) region of the detection antibody. Preferred antibody-binding reagents include protein A, protein G, protein V, protein L, an anti-Fc antibody or antibody-binding fragment and an Fc receptor (FcR) or an antibody-binding fragment thereof. Non-limiting examples of antibodies which can be non-covalently bound to the detection antibody include monoclonal antibodies, polyclonal antibodies, multispecific antibodies, human antibodies, humanized antibodies, chimeric antibodies, single domain antibodies, single chain Fvs (scFv) single chain antibodies, Fab fragments, F(ab') fragments, disulfide-linked Fvs (sdFv), intrabodies, and anti-idiotypic (anti- Id) antibodies, and epitope-binding fragments of any of the above. Non-limiting examples of Fc receptors include FcγRI, FcγRIIA, FcγRIIB, FcγRIIC, FcγRIIIAα, FcγRIIIB, FcεRIα, FcεRIξ and FcγRIIIAξ.

Suitable binding pairs for use in detection include:

- hapten or antigen/antibody, e.g. digoxin and anti-digoxin antibodies
- biotin or biotin analogues (e.g. aminobiotin, iminobiotin or desthiobiotin)/avidin or streptavidin,
- sugar/lectin,
- enzyme and cofactor
- folic acid/folate
- double stranded oligonucleotides that selectively bind to proteins/transcription factors.
- nucleic acid or nucleic acid analogue/complementary nucleic acid,
- receptor/ligand, e.g., steroid hormone receptor/steroid hormone.

It will be understood that the term "first" and "second" member of a binding pair is relative and that each of the above members can be seen as first or second members of the binding pair. In a preferred embodiment, the first member of a binding pair is biotin or a functionally equivalent variant thereof and the second member of the binding pair is avidin, streptavidin or a functionally equivalent variant thereof.

In a preferred embodiment, the second member of the binding pair is streptavidin.

Suitable detectable tags include, without limitation, fluorescent moieties (e.g., fluorescein, rhodamine, phycoerythrin, coumarin, oxazine, resorufin, cyanine and derivatives thereof.), luminescent moieties (e.g., Qdot™ nanoparticles supplied by the Quantum Dot Corporation, Palo Alto, CA).

Suitable substrate-modifying enzymes are those capable of generating a detectable signal, for example, upon addition of an activator, substrate, amplifying agent and the like. Enzymes which are suitable as detectable tags for the present invention and the corresponding substrates include:

- Alkaline phosphatase:

  o Chromogenic substrates: Substrats based on p-nitrophenyl phosphate (p-NPP), 5-bromo-4-chloro-3-indolyl phosphate/nitroblue tetrazolium (BCIP/NPT), Fast-Red/naphthol-AS-TS phosphate
  o Fluorogenic substrates: 4-methylumbelliferyl phosphate (4-MUP), 2-(5'-chloro-2'-phosphoryloxyphenyl)-6-chloro-4-(3H)-quinazolinone (CPPCQ), 3,6-fluorescein diphosphate (3,6-FDP), Fast Blue BB, Fast Red TR, or Fast Red Violet LB diazonium salts

- Peroxidases:

  o Chromogenic substrates based on 2,2-azinobis(3-ethylbenzothiazoline-6-sulfonic acid) (ABTS), o-phenylenediamine (OPT), 3,3',5,5'-tetramethylbenzidine (TMB), o-dianisidine, 5-aminosalicylic acid, 3-dimethylaminobenzoic acid (DMAB) and 3-methyl-2-benzothiazolinehydrazone (MBTH), 3-amino-9-ethylcarbazole (AEC)- and 3,3'-diaminobenzidine tetrahydrochloride (DAB).
  ∘ Fluorogenic susbtrates: 4-hydroxy-3-methoxyphenylacetic acid, reduced phenoxazines and reduced benzothiazines, including Amplex® Red reagent, Amplex UltraRed and reduced dihydroxanthenes.

- Glycosidases:

  o Chromogenic substrates: o-nitrophenyl-β-D-galactoside (o-NPG), p-nitrophenyl-β-D-galactoside and 4- methylumbelliphenyl-β-D-galactoside (MUG) for β-D-galactosidase.
  ∘ Fluorogenic substrates: resorufin β-D-galactopyranoside, fluorescein digalactoside (FDG), fluorescein diglucuronide, 4-methylumbelliferyl β-D-galactopyranoside, carboxyumbelliferyl β-D-galactopyranoside and fluorinated coumarin β-D- galactopyranosides.

- Oxidoreductases (luciferase):

  o Luminiscent substrates: luciferin.

KITS OF THE INVENTION

The invention also provides kits which are suitable for practicing the method of the invention. Thus, in another aspect, the invention provides a kit for determination of amyloid beta peptides in a biological sample comprising

(i) a protein solubilising agent and
(ii) at least an antibody against a amyloid beta peptide.

Components (i) and (ii) of the kit are essentially as described in relation to the method of the invention. In a preferred embodiment, the protein solubilising agent reagent is a detergent. In a still more preferred embodiment, the detergent is Tween20.

In another preferred embodiment, the at least an antibody against the amyloid beta peptide is selected from the group of

(i) an antibody against the N-terminal region of the amyloid beta peptide,
(ii) an antibody against the C-terminal region of the amyloid beta peptide and
(iii) a combination of an antibody against the N-terminal region of the amyloid beta peptide and an antibody against the C-terminal region of the amyloid beta peptide.

In a still more preferred embodiment, the kit of the invention comprises an antibody against the N-terminal region of the amyloid beta peptide which is directed against an epitope located within amino acids 1 to 16 of ABETA40 and ABETA42. In another preferred embodiment, the kit of the invention comprises an antibody against the C-terminal region of the amyloid beta peptide which is directed against a peptide is selected from the group of:

(i) a polyclonal antibody prepared against a peptide corresponding to the C-terminal region of the ABETA42 peptide which binds specifically to ABETA42 without giving any substantial cross-reaction with ABETA40
(ii) a polyclonal antibody prepared against a peptide corresponding to the C-terminal region of the ABETA40 peptide which binds specifically to ABETA40 without giving any substantial cross-reaction with ABETA42 and
(iii) an antibody that recognises simultaneously the C-terminal region of both ABETA40 and ABETA42 and (iv) a combination of the antibodies under (i) and (ii).

In a preferred embodiment, the kit of the invention further comprises a solid support. As used herein, the term "support" or "surface" refers to a solid phase which is a porous or non-porous water insoluble material that can have any one of a number of shapes, such as strip, rod, particles, including latex particles, magnetic particles, microparticles, beads, membranes, microtiter wells and plastic tubes. In principle, any material is suitable as solid support provided that is able to bind sufficient amounts of the capturing antibody. Thus, the choice of solid phase material is determined based upon desired assay format performance characteristics. Materials suitable for the solid support include polymeric materials, particularly cellulosic materials and materials derived from cellulose, such as fibre containing papers, e.g., filter paper, chromatographic paper, glass fiber paper, etc.; synthetic or modified naturally occurring polymers, such as nitrocellulose, cellulose acetate, poly (vinyl chloride), polyacrylamide, cross linked dextrane, agarose, polyacrylate, polyethylene, polypropylene, poly(4-methylbutene), polystyrene, polymethacrylate, poly(ethylene terephthalate), nylon, polyvinyl butyrate), etc.; either used by themselves or in conjunction with other materials; glass such as, e.g., glass available as bioglass, ceramics, metals, and the like. Non-crosslinked polymers of styrene and carboxylated styrene or styrene functionalized with other active groups such as amino, hydroxyl, halo and the like are preferred. In some instances, copolymers of substituted styrenes with dienes such as butadiene will be used.

The solid support and the antibody may be separately provided in the kit or, alternatively, the support may be delivered already precoated with the capture antibody. In this case, the support may have been treated with a blocking solution after the binding of the capture antibody.

Additional components of the kit may include:

• Means for removing from the patient the sample to be analysed.
• Buffers and solutions required for preparing standard curves of the amyloid beta peptides.
• Buffers and solutions for washing and blocking the solid support during the assay
• Buffers and solutions for coating the solid support with the coating antibody
• Reagents for developing the coloured or fluorogenic signal from the detectable tag.
• Reagents for stopping the formation of the coloured or fluorogenic product from the detectable tag (e.g. 1N $H_2SO_4$)
• A sample containing a stock solution of the Aβ40 or Aβ42 peptides or a combination thereof.

In a preferred embodiment, the kit of the invention comprises two antibodies which may be used in an ELISA sandwich assay. In this case, one of the antibodies, the capture antibody, is immobilised onto a solid support. The immobilisation can be carried out prior to the binding of the target polypeptide to be detected or once the peptide/protein is bound to the capture antibody. In any case, if a solid support is used, it is convenient to block the excess of protein binding sites on the carrier prior to the addition of the sample containing the target polypeptide to be determined. Preferably, blocking or quenching of the peptide-binding sites on the support is carried out using the same buffer which is used for washing the complexes after each binding reaction (e.g. 50 mM Tris-HCl, pH 8, PBS or TBS optionally, comprising Tween 20) supplemented with a macromolecular compound (e.g. bovine serum albumin, non-fat dry milk, western blocking reagent, caseine, lactoalbumine, ovoalbumine) in concentrations from about 0.05% to 10%, preferably 1 to 5%, more preferably around 3%.

The invention is described hereinafter by way of the following examples which are to be construed as merely illustrative and not limitative of the scope of the invention.

EXAMPLES

**EXAMPLE 1**

Study population

The study included 40 participants 16 healthy controls (HC), 8 amnesic mild cognitive impairment patients (MCI) and 16 Alzheimer disease patients (AD). All aged over 65 years and 50 % of either sex in each group. Demographic characteristic of the participants are summarized in table 2.

Table 2. Demographic characteristics

| Characteristic | AD | MCI | HC | P[1] |
|---|---|---|---|---|
| Male/Female | 8 / 8 | 4 / 4 | 8 / 8 | |
| Age (years, mean $\pm$ SD) | 78.8 $\pm$ 4.7[H] | 77.3$\pm$3.6[H] | 70.3 $\pm$ 4.1[M,A] | 0.0002 |
| ApoE $\varepsilon$4 frequency (%) | 34.4[H] | 37.5[H] | 3.1[M,A] | 0.002 |
| Education level* | 1.9$\pm$0.9 | 1.6$\pm$0.9 | 2.4$\pm$0.6 | 0.072 |

*Education level is expressed as 0: no studies. 1: Primary Education. 2: Secondary Education. 3: University Education. Kruskal-Wallis U-Test, contrasts with Mann-Whitney U-Test. H, M, and A mean significant with regard to HC, MCI and AD respectively.

Healthy controls were carefully selected among community-dwelling, socially active volunteers with absence of memory complains, normal performance in neuropsychological tests and absence of structural alterations in quantitative magnetic resonance imaging (MRI).

Participants with MCI fulfilled the Mayo Clinic criteria. Additionally for the selection of MCI participants a CDR of 0.5 points, more than 3 points in the in Scheltens (J. Neurol. Neurosurg Psychiatry. 1992; 55:967-972) scale for medial temporal atrophy and a pattern of parietal and/or temporal hypometabolism in positron emission tomography with 18-fluorodeoxyglucose (PET-FDG), suggestive of conversion to AD, was required. Patients with any psychiatric or systemic pathology, other than possible neurodegenerative disease, that could cause the MCI were excluded.

Specific inclusion criteria for the AD group were a diagnosis of probable AD (NINCDS-ADRDA criteria), a CDR of 1 point, a MMSE between 16 and 24 points and more than 3 points in Scheltens scale for medial temporal atrophy.

Cognitive testing for MCI and AD diagnosis was performed according to ACE Memory Clinic routines as described elsewhere.

Written informed consent was obtained from every participant (or in the case of several AD patients by their closest relative). The study protocols were revised and approved by the Ethical Committee of the Hospital Clinic i Provincial (Barcelona, Spain).

**EXAMPLE 2**

Sample preparation

Blood is collected from a subject and a pellet of Roche CompleteMini is added (protease inhibitors) to each of 10 ml. The blood is either directly spun or is preserved at 4°C and is centrifugated on the same day of the assay.

Plasma is separated from the cell fraction and the plasma is collected and split in aliquots of 0.5 ml in Eppendorf tubes. The aliquots can be kept at -80°C.

Free amyloid in plasma (1ab40 and 1ab42) is determined directly in undiluted clarified plasma obtained as explained below. These parameters are hereinafter referred to as 1 ab40 or UP (undiluted plasma) Aβ(1-40) for Aβ(1-40) and as 1ab42 or UP Aβ(1-42) for Aβ(1-42).

Total plasma amyloid, corresponding to the amyloid free in plasma plus the amyloid associated to plasma components, is determined in samples obtained by diluting 150 μl of plasma in 300 μl of dilution buffer (PBS containing 0.5% Tween-20). These parameters are hereinafter referred to as 2ab40 or DP (diluted plasma) Aβ(1-40) for Aβ(1-40) and as 2ab42 or DP Aβ(1-42) for Aβ(1-42).

Cell-bound amyloid beta is determined by diluting the plasma cell fraction v/v 1/5 in dilution sample (PBS containing 0.5% Tween-20). These parameters are hereinafter referred to as 3ab40 or CB (cell bound) Aβ(1-40) for Aβ(1-40) and as 3ab42 or CB Aβ(1-42) for Aβ(1-42).

## EXAMPLE 3

Conditions for sample treatment

*Simple dilution*

In order to identify the dilution of the sample giving the highest absorbance in ELISA assay, different dilutions and buffer solutions were tested.

The samples were diluted ½, 1/3, ¼, 1/5 and 1/10. It was observed that the absorbance of the sample decreased when diluted 1/4 and above. The best dilutions are 1/2 and 1/3.

*Sample centrifugation*

The samples can be clarified by centrifugation for 1' a 13.000 rpm in order to remove particulate components which may interfere with the immunological detection. The supernatant can be collected and tested directly or diluted as described above.

*Sample sonication*

The samples can be sonicated for 5'-10'. The sonicated samples can be used directly in the ELISA assays or can be spinned for 1' at 13000 rpm and the supernatant be used directly in ELISA assays. The sonicated samples can also be diluted using the adequate buffers as defined in the previous examples.

*Sample preclearing*

The samples were run through a Sepharosa 4B-IgGK column in order to remove possible contaminants essentially as desecribed by Fukumoto et al, (Arch. Neurol., 2003, 60: 958-964). Sepharosa 4B-IgGK column was prepared by reacting CNBr-activated sepharose with IgG1k using the following procedure:

- IgGlk (MW=150.000) was dissolved in coupling buffer (0.1M NaHCO$_3$ pH 8.3) and NaCl 0.5M (1.5 ml for 300 mg agarose) using 500 μl IgGlk (0,6 mg) and 1.5 ml Coupling buffer.
- The resin was washed with 1 mM HCl (60 ml for each 300 mg resin)
- The ligand was mixed with the acid-washed resin and incubated overnight at 4ªC with constant shaking (alternatively, the incubation can be carried out for 2 h at room temperature).
- The excess ligand is then washed out using 5 volumes of coupling buffer.
- The unreacted active groups in the resin are then blocked with 0.1 M Tris-HCl pH 8 for 2 h at room temperature under shaking.
- The resin was washed with three cycles using alternatively 0.1M Tris-HCl pH 8 + 0.5M NaCl / 0.1M sodium acetate pH 4 + 0.5M NaCl.

Once the Sepharose 4B-IgGK has been obtained, the treatment of the sample comprises the steps of:

- Contacting 300 μl of plasma with 525 μl sample buffer and 75 μl agarose-IgG1k and incubate for 2h a 4°C con constant stirring.
- The agarose is remover by centrifugation (5' a 1000 rpm)
- 100 μl of the treated sample is added to wells in a microtiter plates onto which a capture antibody specific for the N-terminus of the amyloid beta peptides has been adsorbed and incubated overnight at 4°C.
- The amount of amyloid beta peptide present in the clarified sample is determined by ELISA assay by adding to the wells anti-Aß40 or anti-Aß42 specific antiserum (1/4000) for 1h at room temperature and constant shaking.

- The samples are then incubated with a 1/5000 dilution of a biotynilated anti-rabbit for 1h at room temperature and constant shaking.
- The samples are then incubated with 1/4000 of peroxidase-coupled streptavidin for 1 h at room temperature and shaking
- The reaction was then developed with TMB for 30' in the dark.
- The developing reaction was stopped with stop solution and the absorbance read at 450 nm.

*Albumin and IgG removal*

The samples were run through columns which bind albumin and which bind IgG. The flow through were assayed in order to identify the fraction where the amyloid peptides are found. Albumin is removed using the *"ProteoExtract Albumin Removal, Kit"* (CALBIOCHEM) according to the manufacturer's instructions.

IgG is removed using a protein A column (Protein A Sepharose 4 Fast Flow, Amersham Biosciences) following the manufacturer's instructions.

*Sample concentration*

The samples were concentrated with microcon having a cut-off of 10000. The amyloid peptides are recovered in the flow through whereas high molecular weight proteins are recovered in the retentate.

The effects of the different treatment protocols can be summarized as follows:

- Detergents: Tween-20 is the detergent providing a higher increased in absorbacen values. No effect was observed when the concentration of Tween-20 was increased from 0.05% to 0.1%.
- pH: The pH values providing better results were 7 and 8. When Aß40 was determined, adequate absorbance values were also observed at pH=9. When Aß42 was determined, adequate absorbance values were obtained at pHs 9 and 5.
- Denaturing conditions: The addition of 0.5M or 1 M GuHCl or 10% DMSO did not result in an improvement of the absorbance values.
- Salts: Higher absorbance values were obtained when NaCl was used in comparison with KCl.
- BSA: No differences in absorbance levels were observed when the BSA concentration was increased from 0.05 % to 0.5%.
- Sonication: No differences were observed when the samples were sonicated prior to absorbance determination.
- Preclearing: No effect was observed when the samples were pretrateated with a Sepharosa 4B-IgGk.
- Albumin and IgG removal: Amyloid peptides appear to associate to IgG but not to albumin.
- Concentration: Amyloid peptides appear mainly in the retentate.

## EXAMPLE 4

Colorimetric ELISA sandwich with biotin-streptavidin amplification

In order to increase the sensitivity, the signal can be amplified using biotin-streptavidin. The plate was coated using the 6E10 mAb capture antibody which recognises amino acids 1-17 in both the amyloid Aß40 and in the amyloid Aß42 peptide. The coating was carried out at a concentration of 5 $\mu$g/ml in 100 mM carbonate/bicarbonate buffer, pH=9.6, overnight at 4°C. The plate was then blocked with 300 $\mu$l of a blocking solution (50 mM Tris-HCl, pH 8, 0.2% Tween-20, 0.5% BSA) for 3 h at room temperature with shaking or for 2 h at 37° C. When needed, the plates can be treated, after blocking, with 100 $\mu$l of a 50 mM Tris-HCl pH 8 solution containing 20 mg/ml trehalose. The plates were left to evaporate until a white halo characteristic of trehalose appears. The plates so treated could be kept at 4°C covered with aluminium foil and are stable for two years.

The samples of the standard curve were prepared from a 200 pg/ml stock solution of the peptides Aß40 y Aß42 on plates coated with the 6E10 mAb and treated with trehalose. From these solutions, serial dilutions 1:2 in SDB were made so as to give concentrations of 200, 100, 50, 25, 12.5, 6.25 and 3.125 pg/ml. 100 $\mu$l of each diluted or undiluted sample is added diluted or undiluted in SDB (1/1,000,000) and incubated overnight at 4°C (or for 2h at 37°C). The samples for determining free plasma amyloid, total plasma amyloid and cell bound-amyloid in the test samples are prepared as described in example 1 and added to the wells of the ELISA plates using the same conditions as for the samples of the standard sample. Detection antibody (a polyclonal antibody prepared against a peptide corresponding to the C-terminal region of the A$\beta$42 peptide or a polyclonal antibody prepared against a peptide corresponding to the C-terminal region of the A$\beta$40 peptide, depending on whether A$\beta$42 or A$\beta$40 is to be detected) was added diluted in

SDB. 100 μl are added to each well and were then incubated for 1 h at room temperature. Next, 100 μl of a 1/5000 dilution in SDB of a biotin-labelled anti-rabbit IgG antibody (SIGMA) were then added and incubated for 1h at room temperature with shaking. Then 100 μl of a 1/4000 dilution in SDB of HRP-coupled Streptavidin (from SIGMA) were added to each well and incubated for 1 h at room temperature.

The plate was developed using 100 μl of the chromogenic substrate TMB (ZEU Inmunotec). TMB was added and incubated in the dark during 15-30 minutes. As stop solution, 50 μl of 1N H2SO4 were added per well. The absorbance at 450 nm was read in a plate reader Synergy HT (BioTek Instruments).

The concentration values (pg/ml) obtained from the samples used for the determination of total plasma amyloid (free plasma amyloid together with amyloid bound to the plasma components) was corrected in order to compensate for the dilution carried out during the preparation step. Since the dilution was typically a 1:3 dilution (see Example 1), the pg/ml obtained from the absorbance readouts had to be multiplied by three in order to determine the real aggregate concentration of amyloid free in plasma and amyloid bound to plasma components. Likewise, the pg/ml obtained from the absorbance values obtained from the samples used for the determination of cell-bound amyloid were also corrected in order to compensate for the dilution carried out during the preparation step. Since the dilution was typically 1:5 (see Example 1), the pg/ml obtained from the absorbance readouts had to be multiplied by five in order to determine the real concentration of amyloid bound to cells.

Between each of the steps, the plate was washed using an automatic plate washer (Elx50 Bio Tek Instruments) programmed for performing 5 rinses each time. The washing solution contained 50 mM de Tris-HCl pH 8, 0.05% Tween-20 and 150 mM NaCl (filtered before use).

## EXAMPLE 5

### Fluorescent ELISA Sandwich Assay

The plate was coated with 6E10 in bicarbonate buffer (5 μg/ml) overnight at 4°C. The plate was then blocked 3h at room temperature with shaking (300 μl/well). The test and standard curve samples were then added to the plates and incubated overnight at 4°C. A 1/4000 dilution of the detection antibody (anti-Aß40 o anti-Aß42 serum) was added to each well and incubated for 1h at room temperature with shaking. Serial dilutions of the FITC-coupled anti-antibody (dilutions 1/1000, 1/5000, 1/10000) were added and incubated for 1h at room temperature in the dark. The fluorescence was using an excitation wavelength of 485 nm and an emission wavelength of 528.

Alternatively, the assay is carried out using the *Quanta-Blu* (PIERCE) fluorescent substrate, which increases the sensitivity of the ELISA assay. Maximal excitation is 325 nm and maximal emission is 420 nm. It can be detected in the excitation range of 315-340 nm and 370-470 nm emission range. The QuantaBlu Working Solution is prepared by mixing 9 parts of QuantaBlu Substrate Solution with 1 part of QuantaBlu Stable Peroxidase Solution (solution stable for 24 h at room temperature). It can be incubated from 1.5 minutes to 90 minutes at room temperature and can be read stopping the reaction or without stopping (a blue colour is produced).

The plate is coated with 6E10 mAb in bicarbonate buffer (5 μg/ml) overnight at 4°C and then blocked for 3h at room temperature with shaking (300 μl/well). Different standard curves then prepared with the following concentrations of Aβ42 and Aβ40 peptides:

- 1000, 500, 250, 125, 62.5, 31.25 and 15.65 pg/mL
- 200, 100, 50, 25, 12.5, 6.25 and 3.125 pg/mL
- 25, 12.5, 6.25, 3.125, 1.56, 0.78 and 0.39 pg/mL
- 10, 5, 2.5, 1.25, 0.625, 0.3125 and 0.156 pg/mL
- 5, 2.5, 1.25, 0.625, 0.3125, 0.156 and 0.078 pg/mL
- 1, 0.5, 0.25, 0.125, 0.0625, 0.03125 and 0.0156 pg/mL

The detection antibody (anti-Aß40 o anti-Aß42 serum) is added (diluted at 1/4000) for 1h at room temperature with shaking. The HRP-coupled anti-rabbit IgG 1/1000 is then added and incubated for 1h at room temperature with shaking. For developing the reaction, 100μl of Quanta-Blue Working Solution is added and then incubated for 30', 60' and 90' at room temperature in the darkness. The fluorescence is then read (Excitation: 360/40 nm; Emission: 460/40 nm) at 30', 60' and 90' without stopping the reaction or stopping the reaction with STOP solution.

## EXAMPLE 6

### Preparation of Aβ40 and Aβ42 standard curves

For the preparation of the Aβ40 standard curve, a lyophilised sample of human Aβ40 was reconstituted to 10

μg/mL. From the stock solution, the samples were prepared containing the following concentrations (in pg/mL): 25,000 pg/ml, 2,500 pg/ml, 25 pg/ml, 12.5pg/ml, 6.25 pg/ml, 3.125 pg/ml, 1.56 pg/ml, 0.78 pg/ml. The samples were prepared in the presence of 1 mM of the protease inhibitor AEBSF. The samples were then processed according to the method defined in the previous examples.

For the preparation of the Aβ42 standard curve, a lyophilised sample of human Aß42 was reconstituted to 10 μg/mL. From the stock solution, samples were prepared containing the following concentrations (in pg/mL): 25,000 pg/ml, 2,500 pg/ml, 25 pg/ml, 12.5pg/ml, 6.25 pg/ml, 3.125 pg/ml, 1.56 pg/ml, 0.78 pg/ml. The samples were prepared in the presence of 1 mM of the protease inhibitor AEBSF. The samples were then processed according to the method defined in the previous examples.

## EXAMPLE 7

### Statistical analysis

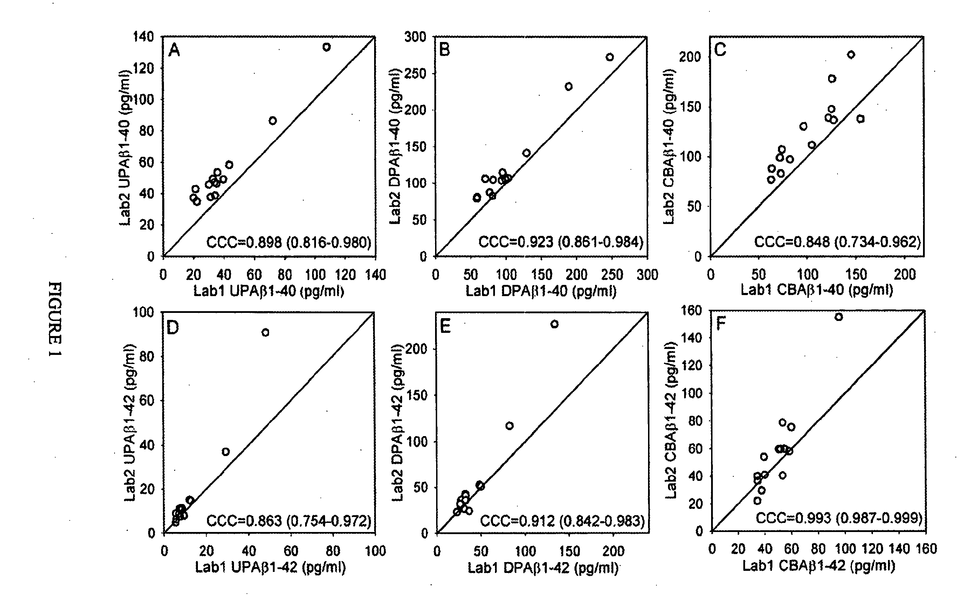

Inter-laboratory reproducibility of the measurements from the 16 randomly chosen samples was assessed by the concordance correlation coefficient (CCC) which evaluates the agreement between the three readings, our own and those reported by the two external laboratories, from the same sample by measuring the variation from the 45 degrees line through the origin (the concordance line). The degree to which samples of the same individual obtained at different days resemble each other (intra-subject reproducibility) was assessed by the intraclass correlation coefficient (ICC). The degree of agreement estimated by these correlation coefficients was described as poor (0.21 to 0.40), moderated (0.41 to 0.60), substantial (0.61 to 0.80) and almost perfect (0.81 to 1.00). The Aβ levels in the different diagnostic groups were compared using Mann-Whitney U-test. Spearman analysis was used to evaluate correlations among continuous variables. For rejection of the null hypothesis a $p < 0.05$ was required. All statistical analysis, including measures of diagnostic accuracy, was performed with SAS 9.1 software. Graphs in Fig. 1 - 3 were generated with PASW statistic software.

The following indicators of the validity and reliability of the amyloid beta peptide markers as a screening test between patients with AD, MCI and HC were estimated.

For each of the markers indicated and determined, the analysis specified below was conducted, classifying the participants among healthy controls-AD, healthy controls-MCI and MCI-AD:

Table 3: Classification of the AD patients versus healthy controls (TP: True positive; FP: False positive; TN: True negative; FN: False negative)

| | | Real classification of the participants | |
|---|---|---|---|
| | | **AD** | **HC** |
| **Classification of the participants according to marker** | **AD** | TP | FP |
| | **HC** | FN | TN |

Table 4: Classification of the MCI patients versus healthy controls (TP: True positive; FP: False positive; TN: True negative; FN: False negative)

| | | Real classification of the participants | |
|---|---|---|---|
| | | **MCI** | **HC** |
| **Classification of the participants according to marker** | **MCI** | TP | FP |
| | **HC** | FN | TN |

Table 5: Classification of the AD patients versus MCI patients (TP: True positive; FP: False positive; TN: True negative; FN: False negative).

| | | Real classification of the participants | |
|---|---|---|---|
| | | AD | MCI |
| Classification of the participants according to marker | AD | TP | FP |
| | MCI | FN | TN |

**Validity properties:**

**Sensitivity:** It is the probability of correctly classifying a sick individual, i.e., the probability of obtaining a positive result for a sick subject in the test. The sensitivity is, therefore, the capacity of the test to detect the disease.

$$Sensitivity = \frac{TP}{TP + FN}$$

**Specificity:** It is the probability of correctly classifying a healthy individual, i.e., the probability of obtaining a negative result for a healthy subject. In other words, specificity can be designed as the capacity to detect healthy people.

$$Specifity = \frac{TN}{TN + FP}$$

**Reliability properties:**

**Positive Predictive Value:** It is the probability of having the disease if a positive result is obtained in the test. The positive predictive value can therefore be estimated from the proportion of patients with a positive result in the test who finally were sick:

$$PPV = \frac{TP}{TP + FP}$$

**Negative Predictive Value:** It is the probability of a subject with a negative result in the test really being healthy. It is estimated by dividing the number of true negatives by the total of patients with a negative result in the test:

$$NPV = \frac{TN}{TN + FN}$$

In addition to the concepts of sensitivity, specificity and predictive values, the concept of likelihood ratio, probability ratio, or odds ratio is also considered. The latter measure the likelier a specific (positive or negative) result is according to the presence or absence of disease.

**Positive likelihood ratio or positive odds ratio:** it is calculated by dividing the probability of a positive result in sick patients by the probability of a positive result among the healthy ones. It is, in short, the ratio between the fraction of true positives (sensitivity) and the fraction of false positives (1-specificity):

$$PLR = \frac{Sensitivity}{1 - Specificity}$$

**Negative likelihood ratio or negative odds ratio:** it is calculated by dividing the probability of a negative result in the presence of disease by the probability of a negative result in the absence thereof. It is therefore calculated as the ratio between the fraction of false negatives (1-sensitivity) and the fraction of true negatives (specificity):

$$NLR = \frac{1 - Sensitivity}{Specificity}$$

The ROC curve is presented by means of a graph, and the area under the ROC curve with 95% CI, the real classification according to the parameter of the (sick/healthy) patients, and the value of sensitivity, specificity, positive predictive value and negative predictive value with 95% CI are presented by means of tables.

All the statistical tests have been performed with a significance level of 0.05.

## EXAMPLE 8

Inter-laboratory reproducibility of Aβ measurements.

Sixteen randomly chosen samples of any participant and extraction were sent to two external laboratories for the evaluation of inter-laboratory reproducibility of the measurements. All markers behaved in a similar way with CCC that ranged from 0.84 to 0.99 (overall 95% IC from 0.73 to 0.99,) which correspond to a degree of agreement between substantial to almost perfect in all cases (Fig. 1).

Average intra-assay reproducibility, expressed as the coefficient of variation of the triplicate wells, for the six markers in each laboratory was 4.31, 5.83 and 8.34 (table 6). The limit of detections of the assays in the three laboratories, were 5.31, 3.63 and 1.91 pg/ml for Aβ1-40 and 2.37, 2.04 and 2.45 pg/ml for Aβ1-42.

Table 6. Intra-assay reproducibility.

|  | LAB1 | | | LAB2 | | | LAB3 | | |
|---|---|---|---|---|---|---|---|---|---|
| MARKER | N | CV | SD | N | CV | SD | N | CV | SD |
| UP Aβ1-40 | 15 | 5.86 | 4.25 | 15 | 5.51 | 4.19 | 16 | 9.87 | 7.21 |
| DP Aβ1-40 | 15 | 2.89 | 1.63 | 15 | 6.97 | 11.58 | 15 | 4.55 | 3.99 |
| CB Aβ1-40 | 15 | 3.10 | 1.44 | 14 | 4.85 | 3.32 | 16 | 8.97 | 4.35 |
| UP Aβ1-42 | 15 | 5.42 | 5.13 | 13 | 5.23 | 4.08 | 16 | 6.33 | 5.75 |
| DP Aβ1-42 | 15 | 3.46 | 2.00 | 15 | 6.61 | 6.90 | 15 | 8.72 | 6.82 |
| CB Aβ1-42 | 15 | 5.11 | 3.60 | 13 | 5.83 | 4.72 | 16 | 11.60 | 14.22 |
| Mean LAB | | 4.31 | 3.01 | | 5.83 | 5.80 | | 8.34 | 7.06 |

CV: Coefficients of variation of the triplicate wells for each marker.

## EXAMPLE 9

Intra-individual reproducibility of Aβ measurements.

The reproducibility of Aβ measurements along the four weekly blood collection (BS1-BS4) as measured by the ICC varied between substantial to almost perfect for all the direct markers in the three groups (table 7). On average for the three diagnostic groups the higher ICC correspond to the measurements of Aβ1-40 and Aβ1-42 in DP (0.93, 95% CI = 0.98 - 0.80 and 0.93, 95% CI = 0.98 - 0.78; respectively).

Table 7. Intra-individual reproducibility.

|  | AD | | | MCI | | | HC | | | Mean Value | | |
|---|---|---|---|---|---|---|---|---|---|---|---|---|
|  | Mean | Max. | Min. | Mean | Max. | Min. | Mean | Max. | Min. | Mean | Max. | Min. |
| UP Aβ1-40 | 0.97 | 0.99 | 0.91 | 0.93 | 0.99 | 0.69 | 0.77 | 0.91 | 0.47 | 0.89 | 0.96 | 0.69 |
| DP Aβ1-40 | 0.97 | 0.99 | 0.92 | 0.95 | 0.99 | 0.77 | 0.88 | 0.96 | 0.71 | 0.93 | 0.98 | 0.80 |
| CB Aβ1-40 | 0.79 | 0.92 | 0.50 | 0.87 | 0.97 | 0.47 | 0.75 | 0.90 | 0.43 | 0.80 | 0.93 | 0.47 |
| UP Aβ1-42 | 0.97 | 0.99 | 0.90 | 0.91 | 0.98 | 0.63 | 0.84 | 0.94 | 0.62 | 0.91 | 0.97 | 0.72 |
| DP Aβ1-42 | 0.98 | 1.00 | 0.96 | 0.91 | 0.98 | 0.61 | 0.91 | 0.97 | 0.77 | 0.93 | 0.98 | 0.78 |
| CB Aβ1-42 | 0.84 | 0.94 | 0.61 | 0.84 | 0.97 | 0.42 | 0.79 | 0.92 | 0.52 | 0.82 | 0.95 | 0.52 |

(continued)

| | AD | | | MCI | | | HC | | | Mean Value | | |
|---|---|---|---|---|---|---|---|---|---|---|---|---|
| | Mean | Max. | Min. | Mean | Max. | Min. | Mean | Max. | Min. | Mean | Max. | Min. |
| Group Mean | 0.92 | 0.97 | 0.80 | 0.90 | 0.98 | 0.60 | 0.82 | 0.93 | 0.59 | 0.88 | 0.96 | 0.66 |

Mean ICC values after comparing the four extractions with each other. All correlations were statistically significant. Max and Min refers to the 95 % confidence intervals.

## EXAMPLE 10

Comparison between diagnostic groups.

In concordance with the high intra-subject reproducibility of the measurements, comparison between groups of participants followed the same pattern in the four blood samples collected at different days (BS1 to BS4) though some p-values vary slightly from one BS to other. The following description is based in the measurements of BS4.

The first striking result was that the concentration of A$\beta$1-40 and A$\beta$1-42 measured in UP represented only around 1/3 and 1/4, respectively, of the levels in DP for any diagnostic groups (table 8).

Table 8. Levels of direct and calculated markers in each group of participants.

| MARKER | AD | | | | MCI | | | | HC | | | |
|---|---|---|---|---|---|---|---|---|---|---|---|---|
| | n | Mean | CV | Range | n | Mean | CV | Range | n | Mean | CV | Range |
| DIRECT | | | | | | | | | | | | |
| UP Aβ1-40 | 15 | 32.2 | 152.5 | 7.2; 203.2 | 7 | 44.7 $^H$ | 102.7 | 14.4;133.6 | 16 | 15.4$^M$ | 47.4 | 2.2;33.3 |
| DP Aβ1-40 | 15 | 115.4 | 137.3 | 12.0; 645.7 | 7 | 124.6 H | 86.9 | 54.5;339.6 | 16 | 56.4$^M$ | 36.2 | 21.4;104.3 |
| CB Aβ1-40 | 15 | 103.3 | 88.2 | 15.3; 328.6 | 7 | 107.4 H* | 54.7 | 63.7;211.2 | 16 | 59.3$^{M*}$ | 28.8 | 14.5;89.2 |
| UP Aβ1-42 | 15 | 23.3 | 206.0 | 4.5; 195.3 | 7 | 24.8H* | 96.8 | 8.6;67.4 | 16 | 8.0$^{M*}$ | 38.7 | 3.7;16.9 |
| DP Aβ1-42 | 15 | 96.5 | 183.3 | 22.3; 728.5 | 7 | 75.2$^H$ | 66.5 | 34.9;151.5 | 16 | 40.1$^M$ | 32.2 | 20.5;78.6 |
| CB Aβ1-42 | 15 | 89.8$^H$ | 59.0 | 52.6;262.6 | 7 | 79.8 | 35.5 | 62.9;141.9 | 16 | 58.7$^A$ | 23.2 | 28.4;76.8 |
| CALCULATED | | | | | | | | | | | | |
| UP Aβ42/Aβ40 | 15 | 0.6 | 50.0 | 0.3;1.2 | 7 | 0.6 | 16.7 | 0.5;0.7 | 16 | 0.7 | 85.7 | 0.1;2.9 |
| DP Aβ42/Aβ40 | 15 | 0.9 | 55.5 | 0.4;2.6 | 7 | 0.7 | 14.3 | 0.4;0.9 | 16 | 0.8 | 25.0 | 0.3;1.5 |
| CB Aβ42/Aβ40 | 15 | 1.2 | 66.7 | 0.4;3.8 | 7 | 0.8 | 25.0 | 0.4;1.1 | 16 | 1.1 | 63.6 | 0.4;3.7 |
| T40 | 15 | 218.8 | 109.8 | 27.3;946.5 | 7 | 232.0 H | 71.7 | 118.2;550.8 | 16 | 115.7 M | 29.2 | 35.9;175.4 |
| T42 | 15 | 186.3$^H$ | 122.0 | 74.9;991.0 | 7 | 155.0 H | 48.3 | 103.9;293.4 | 16 | 98.8$^A$, M | 24.7 | 59.7;155.5 |
| T-βAPB | 15 | 405.1 | 113.1 | 116.6;1937 | 7 | 387.0 H | 60.1 | 222.8;778 | 16 | 214.5 M | 22.1 | 121.8;307.2 |

All values are expressed in pg/ml. H, M, and A mean significant (p<0.05) with regard to HC, MCI and AD respectively. * means p <0.01.

27

Secondly, the CB peptide levels, directly measured from the cellular fraction of the blood sample, were similar to the levels measured in the DP. Moreover, levels of Aβ1-40 and Aβ1-42 strongly correlated when measured in either UP, DP or CB (r = 0.58, 0.71 and 0.71, respectively; p < 0.001). Significant correlations were also found between any pair of the six markers directly assayed in the samples (Aβ1-40 and Aβ1-42 in UP, PD, CB; table 9).

Table 9. Correlation between variables.

| | UP Aβ1-40 | DP Aβ1-40 | CB Aβ1-40 | UP Aβ1-42 | DP Aβ1-42 | CB Aβ1-42 | MMSE | Right MTA |
|---|---|---|---|---|---|---|---|---|
| DP Aβ1-40 | 0.935*** | - | - | - | - | - | - | - |
| CB Aβ1-40 | 0.685*** | 0.776*** | - | - | - | - | - | - |
| UP Aβ1-42 | 0.583*** | 0.556*** | 0.510** | - | - | - | - | - |
| DP Aβ1-42 | 0.652*** | 0.717*** | 0.656*** | 0.806*** | - | - | - | - |
| CB Aβ1-42 | 0.379* | 0.465" | 0.712*** | 0.578*** | 0.693*** | - | - | - |
| MMSE | -0.417** | -0.395* | -0.214 | -0.485** | -0.450** | -0.274 | - | - |
| Right MTA | 0.321* | 0.280 | 0.257 | 0.530** | 0.510** | 0.353* | -0.756*** | - |
| Left MTA | 0.198 | 0.187 | 0.192 | 0.442** | 0.426** | 0.310 | -0.868*** | 0.894*** |

Spearman coefficient for each pair of variables. ***, ** and * mean p < 0.001, 0.01 and 0.05, respectively.

Furthermore, we found that levels of every marker increased in MCI and AD patients with regard to the healthy control group (Fig. 2, table 8). These increments reached statistical significance between the MCI and HC groups for the three Aβ1-40 markers (UP, DP and CB which increased 2.9, 2.2 and 1.8 times, respectively) and for the two Aβ1-42 plasma markers (UP and DP which increased 3.1 and 1.8 times, respectively). Average level of every marker in the AD group was very similar to its average level in the MCI group and not significant differences occurred between these two groups of patients. Similarly, no statistical differences were found between AD and HC groups with the exception of CB levels of Aβ1-42 (Fig. 2). This lack of significance was most probably due to the large range of individual measurements within the AD group (n = 16) which showed CV 1.5 to 2.7 times greater than the MCI (n = 8) group and 2.5 to 5.7 times greater than the HC group (n= 16) for every marker (table 8).

Both Aβ1-40 and Aβ1-42 plasma markers (UP and DP) but not CB, correlated significantly with MMSE (table 9) thought it could be in part overestimated because of the clustering of HC toward the higher punctuations. In fact, excluding participants with MMSE ≥ 26, levels of Aβ1-40 and Aβ1-42 were lower in severely affected patients (MMSE ≤ 21, n = 5) than in moderately affected (MMSE 22-25, n = 12) though differences did not reach statistical significance (data not shown). Additionally, the three Aβ1-42 markers, but not Aβ1-40, were found to significantly correlate with the medial temporal atrophy degree in both the right and left hemisphere (table 9).

We considered as well several markers calculated from those directly assayed in the samples. Apart from the usual Aβ1-42/Aβ1-40 ratios, the most interesting were the sum of DP plus CB Aβ1-40 and the sum of DP plus CB Aβ1-42, which we called total Aβ1-40 (T40) and total Aβ1-42 (T42), respectively and the sum of these two, which we called total βAPB (T-βAPB). The Aβ1-42/Aβ1-40 ratios either measured in UP, DP or CB did not show significant differences between groups. However, the T40, T42 and T-βAPB increased 2.0, 1.5 and 1.8 times, respectively in MCI group with regard to healthy control group (p ≤ 0.03) (table 4). Similar average increments were found between HC and AD patients but in this case only T42 reach statistical significance (Fig. 2).

**EXAMPLE 11**

Diagnostic features of the direct and calculated markers

The direct and calculated parameters mentioned in Table 10 were determined using the methods defined in the previous examples.

Table 10: List of direct and calculated parameters which were analyzed during of the study.

| Direct parameters | |
|---|---|
| *Aβ40* | *Aβ42* |
| 1ab40 (UP) | 1ab42 (UP) |
| 2ab40 (DP) | 2ab42 (DP) |
| 3ab40 (CB) | 3ab42 (CB) |

(continued)

| Calculated parameters | |
|---|---|
| *Sums between Aβ40* | *Sums between Aβ42* |
| 1ab40 + 2ab40<br>1ab40 + 3ab40<br>2ab40 + 3ab40/T40<br>1ab40 + 2ab40 + 3ab40 | 1ab42 + 2ab42<br>1ab42 + 3ab42<br>2ab42 + 3ab42/T42<br>1ab42 + 2ab42 + 3ab42 |
| *Sums between Aβ40 and Aβ42* | |
| 1ab40 + 2ab40 + 1ab42 + 2ab42<br>1ab40 + 3ab40 + 1ab42 + 3ab42<br>2ab40 + 3ab40 + 2ab42 + 3ab42/T-βAPB<br>1ab40 + 2ab40 + 3ab40 + 1ab42 + 2ab42 + 3ab42 | |

The predictive value of the above parameters was tested for

(i) the diagnosis of Alzheimer's disease (by comparing samples from healthy patients with samples from Alzheimer's disease patients, AD/HC),

(ii) the diagnosis of mild cognitive impairment and the stage prior to Alzheimer's disease (by comparing samples from healthy patients with samples from patients suffering mild cognitive impairment, MCI/HC) and

(iii) distinguishing mild cognitive impairment from Alzheimer's disease (by comparing samples from patients suffering mild cognitive impairment with samples from Alzheimer's disease patients, MCI/AD).

Diagnostic characteristics of the assays were assessed by logistic analysis of Aβ measurements and clinical diagnosis considered as the gold standard. The results regarding sensitivity, specificity, positive predictive value (PPV), negative predictive value (NPV), accuracy and area under the Receiver Operating Characteristic (ROC) curve are summarized in table 11.

Table 11. Diagnostic features of direct and calculated βAPB markers.

| Marker | Cutoff (pg/ml) | | Sensitivity (> 85%) | Specificity (> 75%) | PPV | NPV | Accuracy (> 80 %) | ROC (> 0.8) |
|---|---|---|---|---|---|---|---|---|
| UP Aβ1-40 (1ab40) | 23.2 AD | AD/HC | 40.0 | 93.8 | 85.7 | 62.5 | 67.7 | 0.6 |
| | 17.0 MCI | MCI/HC | 85.7 | 68.8 | 54.5 | 91.7 | 73.9 | 0.8 |
| | 22.5 AD | AD/MCI | 40.0 | 71.4 | 75.0 | 35.7 | 50.0 | 0.4 |
| DP Aβ1-40 (2ab40) | 63.8 AD | AD/HC | 46.7 | 81.3 | 70.0 | 61.9 | 64.5 | 0.6 |
| | 63.8 MCI | MCI/HC | 85.7 | 81.3 | 66.7 | 92.9 | 82.6 | 0.8 |
| | 72.2 AD | AD/MCI | 40.0 | 71.4 | 75.0 | 35.7 | 50.0 | 0.4 |
| CB Aβ1-40 (3ab40) | 71.9 AD | AD/HC | 53.3 | 87.5 | 80.0 | 66.7 | 70.9 | 0.7 |
| | 71.1 MCI | MCI/HC | 85.7 | 81.3 | 66.7 | 92.9 | 82.6 | 0.9 |
| | 211.3 AD | AD/MCI | 13.3 | 100.0 | 100.0 | 35.0 | 40.9 | 0.4 |
| UP Aβ1-42 (1ab42) | 10.28 AD | AD/HC | 46.7 | 87.5 | 77.8 | 63.6 | 67.7 | 0.7 |
| | 9.2 MCI | MCI/HC | 85.7 | 81.3 | 66.7 | 92.9 | 82.6 | 0.8 |
| | 67.4 AD | AD/MCI | 6.7 | 100.0 | 100.0 | 33.3 | 36.3 | 0.3 |
| DP Aβ1-42 (2ab42) | 47.4 AD | AD/HC | 46.7 | 87.5 | 77.8 | 63.6 | 67.7 | 0.6 |
| | 50.3 MCI | MCI/HC | 57.1 | 93.8 | 80.0 | 83.3 | 82.6 | 0.8 |
| | 151.7 AD | AD/MCI | 6.7 | 100.0 | 100.0 | 33.3 | 36.3 | 0.4 |
| CB Aβ1-42 (3ab42) | 76.9 AD | AD/HC | 40.0 | 100.0 | 100.0 | 64.0 | 70.9 | 0.7 |
| | 59.8 MCI | MCI/HC | 100.0 | 50.0 | 46.7 | 100.0 | 65.2 | 0.7 |
| | 71.3 AD | AD/MCI | 53.3 | 71.4 | 80.0 | 41.7 | 59.0 | 0.5 |
| T40 (DP + CB) (2ab40 + 3ab40) | 132.7 AD | AD/HC | 53.3 | 81.3 | 72.7 | 65 | 67.7 | 0.6 |
| | 132.7 MCI | MCI/HC | 85.7 | 85.3 | 66.7 | 92.9 | 86.3 | 0.8 |
| | 550.8 AD | AD/MCI | 13.3 | 100 | 100 | 35 | 40 | 0.4 |
| T42 (DP + CB) (2ab42 + 3ab42) | 115.8 AD | AD/HC | 53.3 | 87.5 | 80 | 66.7 | 70.9 | 0.7 |
| | 103.3 MCI | MCI/HC | 100 | 50 | 46.7 | 100 | 68 | 0.8 |

| Marker | Cutoff (pg/ml) | | Sensitivity (> 85%) | Specificity (> 75%) | PPV | NPV | Accuracy (> 80 %) | ROC (> 0.8) |
|---|---|---|---|---|---|---|---|---|
| T-βAPB (T40 + T42) (2ab40 + 3ab40 + 2ab42 + 3ab42) | 113.7 AD | AD/MCI | 53.3 | 57.1 | 72.7 | 36.4 | 54 | 0.4 |
| | 235.5 AD | AD/HC | 53.3 | 81.3 | 72.7 | 65 | 38 | 0.7 |
| | 235.5 MCI | MCI/HC | 85.7 | 81.3 | 66.7 | 92.9 | 86.2 | 0.8 |
| | 778.1 AD | AD/MCI | 13.3 | 100 | 100 | 35 | 40 | 0.4 |

Highlighted in grey are the results that met the criterion considered suitable as figure in the heading. PPV: positive predictive value. NPV: negative predictive value. ROC: area under the receiver operating characteristic curve.

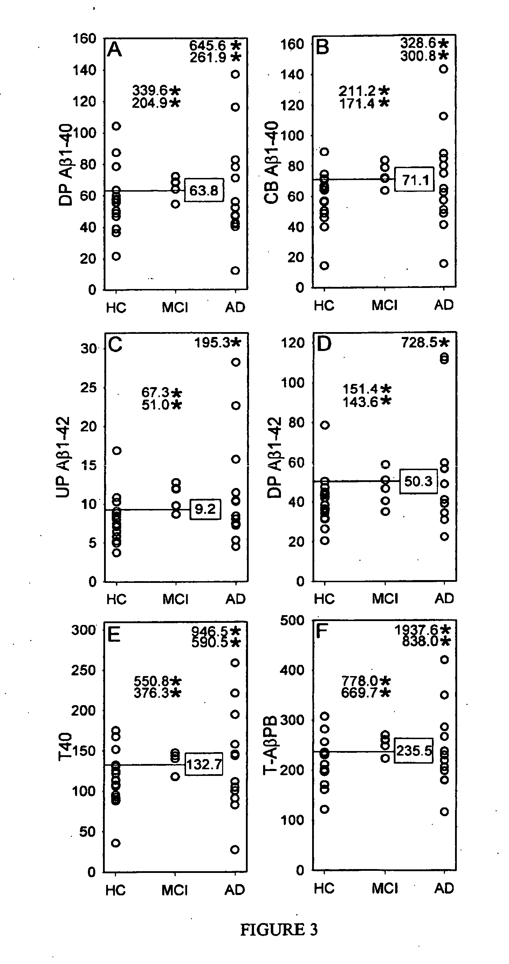

Most direct markers and two calculated markers (T40 and T-βAPB) met the criteria considered suitable to distinguish between MCI patients and HC which is of the utmost interest because from any practical point of view it is here where the diagnostic should be improved. Thus, all the direct markers, except CB Aβ1-42, presented a ROC ≥ 0.8 and among them four (DP Aβ1-40, CB Aβ1-40, UP Aβ1-42 and DP Aβ1-42) got accuracies > 80% which means that 80% of the test were correct when compared with the clinical gold standard (Fig. 3). The calculated T40 and T-βAPB were equally accurate to distinguish between MCI and HC than the direct markers whereas T42 appear to be less reliable (Fig. 3). Due to the great variability of Aβ measurements from one individual to another within the AD group, not any cutting point could be found at which these markers discriminated the AD patients from the other two groups of participants with an acceptable sensitivity and specificity (Fig. 3).

## EXAMPLE 12

Other paramenters showing the highest sensitivity and sensibility and suitable for use in the present invention include those shown in Table 12.

| Method | Parameter |
|---|---|
| Detection of a stage prior to a neurodegenerative disease | DP (Aβ1-40)/2ab40 |
| Diagnosis of a neurodegenerative disease | CB (Aβ1-40)/3ab40 |
| Detection of a stage prior to a neurodegenerative disease | CB (Aβ1-40)/3ab40 |
| Distinguishing a neurodegenerative disease from a stage prior to said neurodegenerative disease | CB (Aβ1-40)/3ab40 |
| Diagnosis of a neurodegenerative disease | DP (Aβ1-42)/2ab42 |
| Detection of a stage prior to a neurodegenerative disease | DP (Aβ1-42)/2ab42 |
| Distinguishing a neurodegenerative disease from a stage prior to said neurodegenerative disease | DP (Aβ1-42)/2ab42 |
| Diagnosis of a neurodegenerative disease | CB (Aβ1-42)/3ab42 |
| Detection of a stage prior to a neurodegenerative disease | CB (Aβ1-42)/3ab42 |
| Diagnosis of a neurodegenerative disease | DP (Aβ1-40) + CB (Aβ1-40)/ 2ab40 + 3ab40 |
| Detection of a stage prior to a neurodegenerative disease | DP (Aβ1-40) + CB (Aβ1-40)/ 2ab40 + 3ab40 |
| Distinguishing a neurodegenerative disease from a stage prior to said neurodegenerative disease | DP (Aβ1-40) + CB (Aβ1-40)/ 2ab40 + 3ab40 |
| Diagnosis of a neurodegenerative disease | DP (Aβ1-42) + CB (Aβ1-42)/ 2ab42 + 3ab42 |
| Detection of a stage prior to a neurodegenerative disease | DP (Aβ1-42) + CB (Aβ1-42)/ 2ab42 + 3ab42 |
| Diagnosis of a neurodegenerative disease | DP (Aβ1-40) + CB (Aβ1-40) + DP (Aβ1-42) + CB (Aβ1-42)/ 2ab40 + 3ab40 + 2ab42 + 3ab42 |
| Detection of a stage prior to a neurodegenerative disease | DP (Aβ1-40) + CB (Aβ1-40) + DP (Aβ1-42) + CB (Aβ1-42)/ 2ab40 + 3ab40 + 2ab42 + 3ab42 |
| Distinguishing a neurodegenerative disease from a stage prior to said neurodegenerative disease | DP (Aβ1-40) + CB (Aβ1-40) + DP (Aβ1-42) + CB (Aβ1-42)/ 2ab40 + 3ab40 + 2ab42 + 3ab42 |
| Detection of a stage prior to a neurodegenerative disease | FP (Aβ1-40) + DP (Aβ1-40) + CB (Aβ1-40) + FP (Aβ1-42) + DP (Aβ1-42) + CB (Aβ1-42)/ 1ab40 + 2ab40 + 3ab40 + 1ab42 + 2ab42 + 3ab42 |
| Detection of a stage prior to a neurodegenerative disease | FP (Aβ1-40) + DP (Aβ1-40) + CB (Aβ1-40)/ 1ab40 + 2ab40 + 3ab40 |
| Detection of a stage prior to a neurodegenerative disease | FP (Aβ1-42) + DP (Aβ1-42) + CB (Aβ1-42)/ 1ab42 + 2ab42 + 3ab42 |
| Diagnosis of a neurodegenerative disease | FP (Aβ1-40) + DP (Aβ1-40) FP (Aβ1-42) + DP (Aβ1-42) / 1ab40 + 2ab40 + 1ab42 + 2ab42 |
| Detection of a stage prior to a neurodegenerative disease | FP (Aβ1-40) + DP (Aβ1-40) FP (Aβ1-42) + DP (Aβ1-42) / 1ab40 + 2ab40 + 1ab42 + 2ab42 |
| Distinguishing a neurodegenerative disease from a stage | FP (Aβ1-40) + DP (Aβ1-40) FP |

| prior to said neurodegenerative disease | (Aβ1-42) + DP (Aβ1-42) / 1ab40 + 2ab40 + 1ab42 + 2ab42 |
|---|---|
| Detection of a stage prior to a neurodegenerative disease | FP (Aβ1-40) + CB (Aβ1-40) FP (Aβ1-42) + CB (Aβ1-42) / 1ab40 + 3ab40 + 1ab42 + 3ab42 |

Table 12: Summary of methods showing better sensitivity, specificity and accuracy levels. The most preferred methods are given in white characters on a black background. The second best methods are shown using a light grey background.

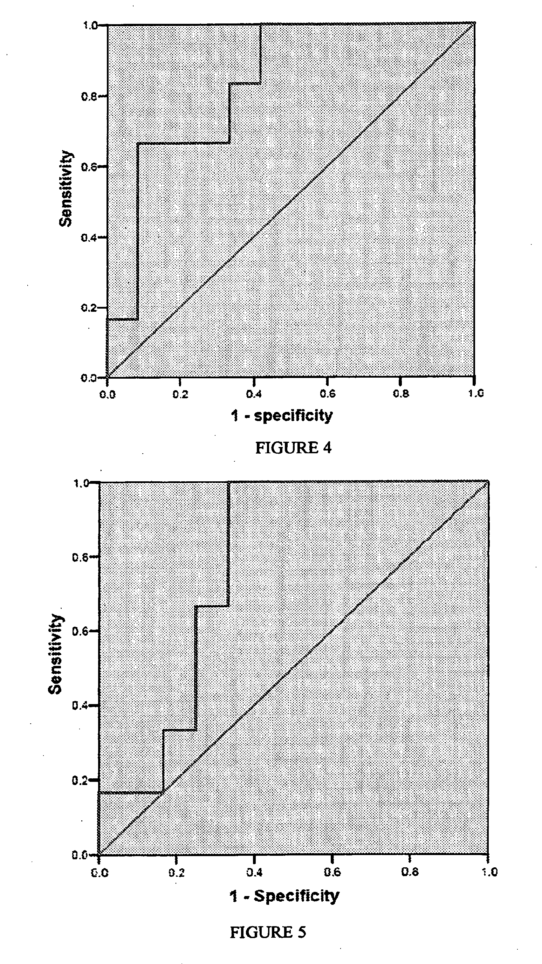

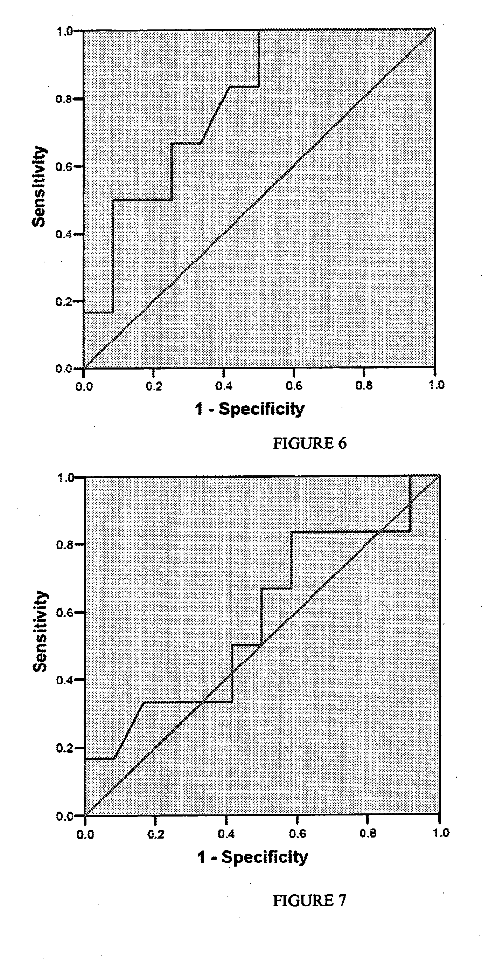

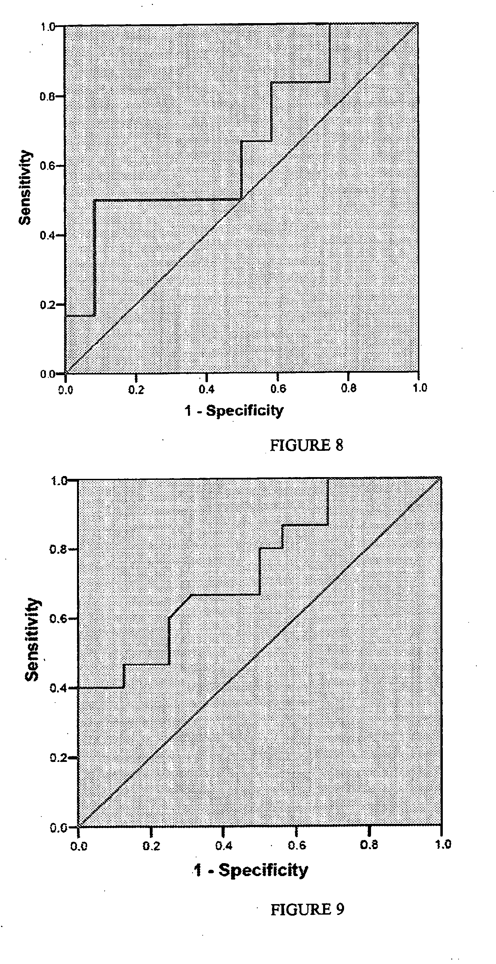

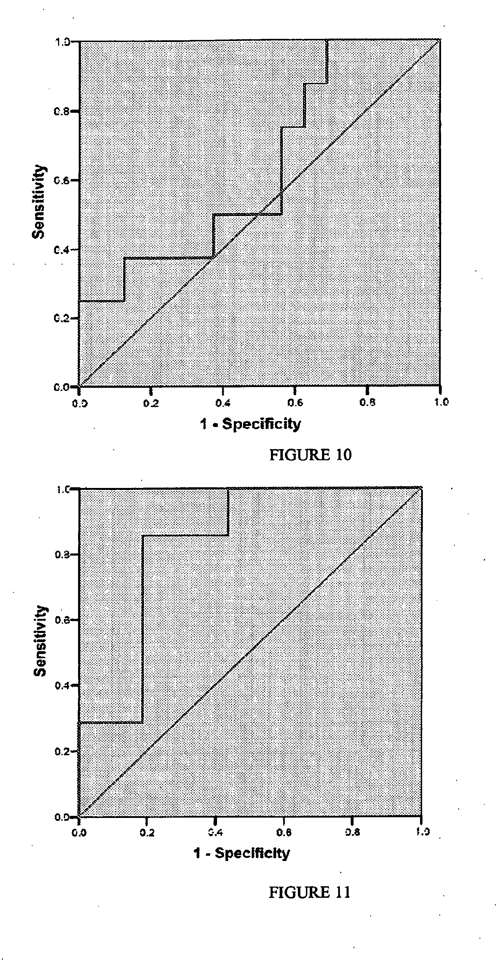

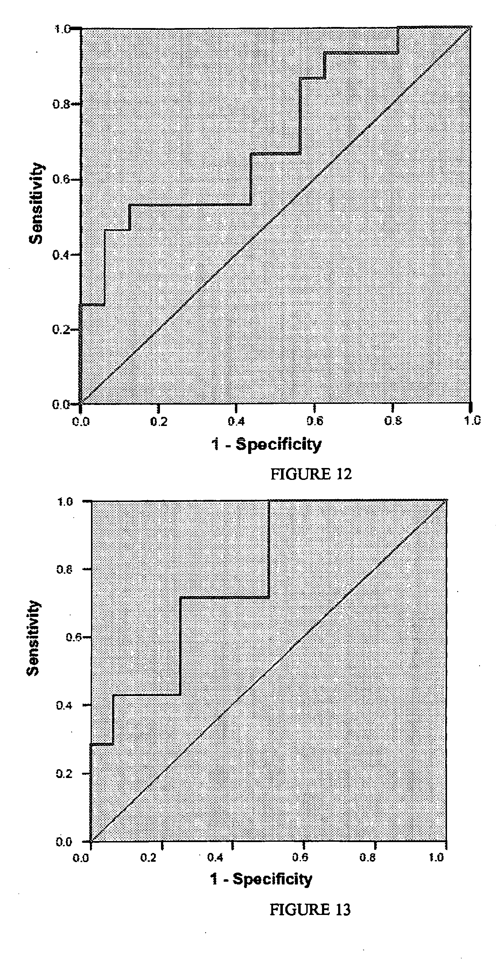

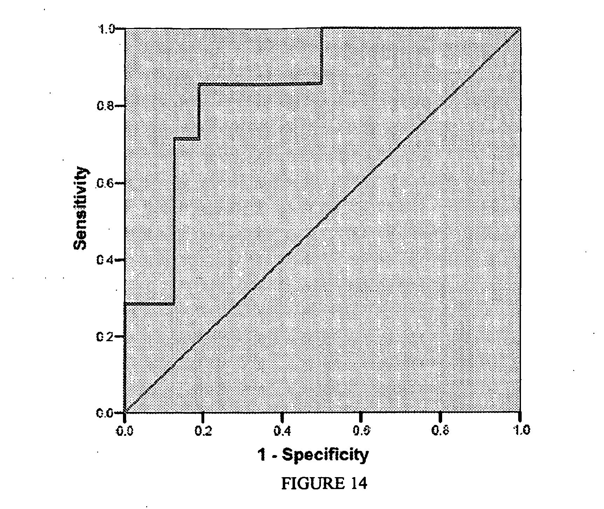

The methods provided particularly useful for detecting mild cognitive impairment from healthy patients using the 1ab40 marker (figure 4), the 2ab40 marker (figure 5), the 3ab40 marker (figure 6), the 1ab42 marker (figure 7), the 2ab42 marker (figure 8), the 3ab42 marker (figures 9 and 10), the 2ab40 + 3ab40 marker (figure 11), the 2ab42 + 3ab42 marker (figure 12), the 2ab42 + 3ab42 (figure 13) and the 2ab40 + 3ab40 + 2ab42 + 3ab42 (figure 14).

## Claims

1. A method for the diagnosis of a neurodegenerative disease in a subject, for detecting a stage prior to a neurodegenerative disease or for distinguishing a neurodegenerative disease from a stage prior to said neurodegenerative disease comprising the steps of

   (i) determining one or more parameters selected from the group of

   (a) the level of one or more free amyloid beta peptides in a biological sample of said subject,
   (b) the aggregate levels of a one or more free amyloid peptides in a biological sample of said subject and of-said one or more amyloid beta peptides associated to macromolecular components present in said biological sample, wherein said aggregate levels are determined by quantifying the amount of said one or more amyloid beta peptides in cell-free fraction of said sample after contacting said sample with a protein solubilising agent under conditions adequate to promote dissociation of the amyloid beta peptide or peptides from the components present in the biological sample,
   (c) the level of one or more amyloid beta peptides associated to cells in a biological sample of said subject, wherein said level is determined by isolating the cell fraction of said biological sample, contacting said cellular fraction of said sample with a protein solubilising agent under conditions adequate to promote dissociation of the amyloid beta peptide or peptides from the cells present in the sample and

   (ii) comparing the value of at least one of the parameters (b) or (c) or the value of a calculated parameter resulting from arithmetically combining at least two of the parameters (a) to (c) with a reference value corresponding to the value of said parameters (b) or (c) or said calculated parameter in a reference sample and
   (iii) diagnosing the neurodegenerative disease, detecting a stage prior to a neurodegenerative disease or distinguishing a neurodegenerative disease from a stage prior to said neurodegenerative disease when there is an alteration in the value of the parameter or in the value of the calculated parameter with respect to the reference value.

   wherein the biological sample is plasma or blood, and wherein the parameters determined in step (i) are one or more of the parameters selected from the group of

   (a) 1ab40, corresponding to the level of free ABETA40 peptide in a biological sample of said subject,
   (b) 1ab42, corresponding to the level of free ABETA42 peptide in a biological sample of said subject,
   (c) 2ab40, corresponding to the aggregate levels of free ABETA40 peptide in a biological sample of said subject and of ABETA40 peptide associated to components of said biological sample, wherein 2ab40 is determined by quantifying the amount of ABETA40 peptide in said sample after contacting said sample with a protein solubilising agent under conditions adequate to promote dissociation of the ABETA40 peptide from the components present in the biological sample,

(d) 2ab42, corresponding to the aggregate level of free ABETA42 peptide in a biological sample of said subject and of ABETA42 peptide associated to components of said biological sample, wherein 2ab42 is determined by quantifying the amount of ABETA42 peptide in said sample after contacting said sample with a protein solubilising agent under conditions adequate to promote dissociation of the ABETA42 peptide from the components present in the biological sample,

(e) 3ab40, corresponding to the level of the ABETA40 peptide associated to cells in a biological sample of said subject, wherein 3ab40 is determined by quantifying the amount of ABETA40 peptide after contacting the cellular fraction of said biological sample with a protein solubilising agent under conditions adequate to promote dissociation of the amyloid beta peptides from the cells present in the sample and

(f) 3ab42, corresponding to the level of the ABETA42 peptide associated to cells in a biological sample of said subject, wherein 3ab42 is determined by quantifying the amount of ABETA42 peptide after contacting the cellular fraction of said biological sample with a protein solubilising agent under conditions adequate to promote dissociation of the amyloid beta peptides from the cells present in the sample.

2. A method as defined in claim 1 wherein the calculated parameter obtained in step (ii) is selected from the group of 2ab40/2ab42, 3ab40/3ab42, 2ab40/3ab40, 2ab42/3ab42, 1ab40 + 2ab40, 1ab40 + 3ab40, 2ab40 + 3ab40, 1ab40 + 2ab40 + 3ab40, 1ab42 + 2ab42, 1ab42 + 3ab42, 2ab42 + 3ab42, 1ab42 + 2ab42 + 3ab42, 1ab40 + 2ab40 + 1ab42 + 2ab42, 1ab40 + 3ab40 + 1ab42 + 3ab42, 2ab40 + 3ab40 + 2ab42 + 3ab42, 1ab40 + 2ab40 + 3ab40 + 1ab42 + 2ab42 + 3ab42, (1ab40 + 2ab40)/(1ab42 + 2ab42), (1ab40 + 3ab40)/(1ab42 + 3ab42), (2ab40 + 3ab40)/(2ab42 + 3ab42), (1ab40 + 2ab40 + 3ab40)/(1ab42 + 2ab42 + 3ab42), (1ab42 + 2ab42)/(1ab40 + 2ab40), (1ab42 + 3ab42)/(1ab40 + 3ab40), (2ab42 + 3ab42)/(2ab40 + 3ab40), (1ab42 + 2ab42 + 3ab42)/(1ab40 + 2ab40 + 3ab40), 2ab40-1ab40, 2ab42-1ab42 and (2ab40-1ab40)/(2ab42-1ab42).

3. A method as defined in claims 1 or 2 wherein

(i) the diagnosis of a neurodegenerative disease is carried out by comparing the value of a parameter selected from the group of 3ab40, 2ab42 and 3ab42 or the value of a calculated parameter selected from the group of 2ab40 + 3ab40, 2ab42+3ab42, 1ab40+2ab40+1ab42+2ab42 and 2ab40 + 3ab40 + 2ab42 + 3ab42,

(ii) the detection of a stage prior to a neurodegenerative disease is carried out by comparing the value of a parameter selected from the group of 2ab40, 3ab40, 2ab42 and 3ab42 or the value of a calculated parameter selected from the group of 2ab40 + 3ab40, 2ab42 + 3ab42, 2ab40 + 3ab40 + 2ab42 + 3ab42, 1ab40 + 2ab40 + 3ab40 + 1ab42 + 2ab42 + 3ab42, 1ab40 + 2ab40 + 3ab40, 1ab42 + 2ab42 + 3ab42, 1ab40 + 2ab40 + 1ab42 + 2ab42 and 1ab40 + 3ab40 + 1ab42 + 3ab42 or

(iii) the distinguishing of a neurodegenerative disease from a stage prior to said neurodegenerative disease is carried out by comparing the value of a parameter selected from the group of 3ab40 and 2ab42 or by comparing the value of a calculated parameter selected from the group of 2ab40 + 3ab40, 2ab40 + 3ab40 + 2ab42 + 3ab42 and 1ab40 + 2ab40 + 1ab42 + 2ab42.

4. A method as defined in claim 3 wherein the reference value used in step (ii) to compare the value of the parameter or the value of the calculated parameter is selected from the group of

(i) 63.8 pg/ml when the parameter 2ab40 is used for the detection of a stage prior to a neurodegenerative disease,

(ii) 71.9 pg/ml when the parameter 3ab40 is used for the diagnosis of a neurodegenerative disease,

(iii) 71.1 pg/ml when the parameter 3ab40 is used for the detection of a stage prior to a neurodegenerative disease,

(iv) 211.3 pg/ml when the parameter 3ab40 is used for distinguishing a neurodegenerative disease from a stage prior to said neurodegenerative disease,

(v) 47.4 pg/ml when the parameter 2ab42 is used for the diagnosis of a neurodegenerative disease,

(vi) 50.3 pg/ml when the parameter 2ab42 is used for the detection of a stage prior to a neurodegenerative disease,

(vii) 151.7 pg/ml when the parameter 2ab42 is used for distinguishing a neurodegenerative disease from a stage prior to said neurodegenerative disease,

(viii) 76.9 pg/ml when the parameter is 3ab42 and is used for the diagnosis of a neurodegenerative disease,

(ix) 58.8 pg/ml when the parameter is 3ab42 and used for the detection o a stage prior to a neurodegenerative disease,

(x) 132.7 pg/ml when the parameter 2ab40 + 3ab40 is used for the diagnosis of a neurodegenerative disease,

(xi) 132.7 pg/ml when the parameter 2ab40 + 3ab40 is used for the detection of a stage prior to a neurodegenerative disease,

(xii) 550.8 pg/ml when the parameter 2ab40 + 3ab40 is used for distinguishing a neurodegenerative disease from a stage prior to said neurodegenerative disease,

(xiii) 115.8 pg/ml when the parameter is 2ab42 + 3ab42 and is used for the diagnosis of a neurodegenerative disease,

(xiv) 103.3 pg/ml when the parameter is 2ab42+3ab42 and is used for the detection of a stage prior to a neurodegenerative disease,

(xv) 235.5 pg/ml when the parameter 2ab40 + 3ab40 + 2ab42 + 3ab42 is used for the for the diagnosis of a neurodegenerative disease,

(xvi) 235.5 pg/ml when the parameter 2ab40 + 3ab40 + 2ab42 + 3ab42 is used for the for the detection of a stage prior to a neurodegenerative disease,

(xvii) 778.1 pg/ml when the parameter 2ab40 + 3ab40 + 2ab42 + 3ab42 is used for for distinguishing a neurodegenerative disease from a stage prior to said neurodegenerative disease,

(xviii) 272.1 pg/ml when the parameter 1ab40 + 2ab40 + 3ab40 + 1ab42 + 2ab42 + 3ab42 is used for the detection of a stage prior to a neurodegenerative disease,

(xix) 155.8 pg/ml when the parameter 1ab40 + 2ab40 + 3ab40 is used for the detection of a stage prior to a neurodegenerative disease,

(xx) 124.3 pg/ml when the parameter 1ab42 + 2ab42 + 3ab42 is used for the detection of a stage prior to a neurodegenerative disease,

(xxi) 158.3 pg/ml when the parameter 1ab40 + 2ab40 + 1ab42 + 2ab42 is used for the diagnosis of a neurodegenerative disease,

(xxii) 142.4 pg/ml when the parameter 1ab40 + 2ab40 + 1ab42 + 2ab42 is used for the detection of a stage prior to a neurodegenerative disease,

(xxiii) 161.2 pg/ml when the parameter 1ab40 + 2ab40 + 1ab42 + 2ab42 is used for distinguishing a neurodegenerative disease from a stage prior to said neurodegenerative disease and

(xxiv) 154.7 pg/ml when the parameter 1ab40 + 3ab40 + 1ab42 + 3ab42 is used for the detection of a stage prior to a neurodegenerative disease.

**5.** A method as defined in any of claims 1 to 4 wherein the alteration in the value of the parameter or in the value of the calculated parameter with respect to the reference value is an increase.

**6.** A method as defined in any of claims 1 to 5 wherein the protein solubilising agent is a detergent, preferably Tween 20.

**7.** A method as defined in any of claims 1 to 6 wherein the determination step of the at least one or more of 1ab40, 1ab42, 2ab40, 2ab42, 3ab40 and 3ab42 is carried out by an immunological method.

**8.** A method as defined in claim 7 wherein said immunological method is an ELISA assay.

**9.** A method as defined in claim 8 wherein the ELISA assay is an ELISA sandwich assay.

**10.** A method as defined in claim 9 wherein the capture antibody in the ELISA sandwich assay is an antibody against the N-terminal region of the amyloid beta peptide.

**11.** A method as defined in claim 10 wherein the antibody against the N-terminal region of the amyloid beta peptide is directed against an epitope located within amino acids 1 to 7 of ABETA40 and ABETA42.

**12.** A method as defined in any of claims 8 to 11 wherein the detection antibody in the ELISA sandwhich assay is an antibody specific against an epitope located in the C-terminal region of the amyloid beta peptide.

**13.** A method as defined in claim 12 wherein the antibody specific against the C-terminal region of the amyloid beta peptide is selected from the group of

(i) a polyclonal antibody prepared against a peptide corresponding to the C-terminal region of the ABETA42 peptide which binds specifically to ABETA42 without giving any substantial cross-reaction with ABETA40,

(ii) a polyclonal antibody prepared against a peptide corresponding to the C-terminal region of the ABETA40 peptide which binds specifically to ABETA40 without giving any substantial cross-reaction with ABETA42 and

(iii) an antibody that recognizes simultaneously the C-terminal region of both ABETA40 and ABETA42 and (iv) a combination of the antibodies under (i) and (ii).

14. A method as defined in any of claims 9 to 13 wherein the detection antibody is further detected using a reagent showing affinity for said antibody which is coupled to a first member of a binding pair.

15. A method as defined in claim 14 wherein the detection is carried out using a second member of a binding pair coupled to a detectable tag.

16. A method as defined in any of claims 1 to 15 wherein the neurodegenerative disease is Alzheimer's disease and/or the stage prior to a neurodegenerative disease is mild cognitive impairment.

**Patentansprüche**

1. Verfahren zur Diagnose einer neurodegenerativen Erkrankung in einem Subjekt, zum Nachweisen eines Stadiums vor einer neurodegenerativen Erkrankung oder zum Unterscheiden einer neurodegenerativen Erkrankung von einem Stadium vor der neurodegenerativen Erkrankung, wobei das Verfahren die folgenden Schritte umfasst:

(i) Bestimmen von ein oder mehreren Parametern, die aus der Gruppe ausgewählt sind, bestehend aus

(a) der Konzentration von ein oder mehreren freien Amyloid-beta-Peptiden in einer biologischen Probe von dem Subjekt,
(b) den Aggregatskonzentrationen von ein oder mehreren freien Amyloid-beta-Peptiden in einer biologischen Probe von dem Subjekt und von ein oder mehreren Amyloid-beta-Peptiden, die mit makromolekularen Bestandteilen, die in der biologischen Probe vorhanden sind, assoziiert sind, wobei die Aggregatskonzentrationen bestimmt werden, indem die Menge an den ein oder mehreren Amyloid-beta-Peptiden in der zellfreien Fraktion der Probe quantifiziert wird, nachdem die Probe mit einem Proteinsolubilisierungsmittel unter Bedingungen in Kontakt gebracht worden ist, die geeignet sind, um die Dissoziation des Amyloid-beta-Peptids oder der Amyloid-beta-Peptide von den Bestandteilen, die in der biologischen Probe vorhanden sind, zu begünstigen, und
(c) der Konzentration von ein oder mehreren Amyloid-beta-Peptiden, die mit Zellen in einer biologischen Probe von dem Subjekt assoziiert sind, wobei die Konzentration bestimmt wird, indem die Zellfraktion von der biologischen Probe isoliert wird und die zelluläre Fraktion der Probe mit einem Proteinsolubilisierungsmittel unter Bedingungen in Kontakt gebracht wird, die geeignet sind, um die Dissoziation des Amyloid-beta-Peptids oder der Amyloid-beta-Peptide von den Zellen, die in der biologischen Probe vorhanden sind, zu begünstigen, und

(ii) Vergleichen des Werts von mindestens einem der Parameter (b) oder (c) oder des Werts von einem berechneten Parameter, der sich aus dem arithmetischen Kombinieren von mindestens zwei der Parameter (a) bis (c) ergibt, mit einem Bezugswert, der dem Wert der Parameter (b) oder (c) oder des berechneten Parameters in einer Bezugsprobe entspricht, und
(iii) Diagnostizieren der neurodegenerativen Erkrankung, Nachweisen eines Stadiums vor einer neurodegenerativen Erkrankung oder Unterscheiden einer neurodegenerativen Erkrankung von einem Stadium vor der neurodegenerativen Erkrankung, wenn es eine Veränderung in dem Wert des Parameters oder in dem Wert des berechneten Parameters in Bezug auf den Bezugswert vorliegt,

wobei die biologische Probe Plasma oder Blut ist und wobei die Parameter, die in dem Schritt (i) bestimmt werden, ein oder mehrere der Parameter sind, die aus der Gruppe ausgewählt sind, bestehend aus:

(a) 1ab40, das der Konzentration von freiem ABETA40-Peptid in einer biologischen Probe von dem Subjekt entspricht,
(b) 1 ab42, das der Konzentration von freiem ABETA42-Peptid in einer biologischen Probe von dem Subjekt entspricht
(c) 2ab40, das den Aggregatskonzentrationen von freiem ABETA40-Peptid in einer biologischen Probe von dem Subjekt und von ABETA40-Peptid, das mit Bestandteilen der biologischen Probe assoziiert ist, entspricht, wobei 2ab40 bestimmt wird, indem die Menge an ABETA40-Peptid in der Probe quantifiziert wird, nachdem die Probe mit einem Proteinsolubilisierungsmittel unter Bedingungen in Kontakt gebracht worden ist, die geeignet sind, um die Dissoziation des ABETA40-Peptids von den Bestandteilen, die in der biologischen Probe vorhanden sind, zu begünstigen,

(d) 2ab42, das der Aggregatskonzentration von freiem ABETA42-Peptid in einer biologischen Probe von dem Subjekt und von ABETA42-Peptid, das mit Bestandteilen der biologischen Probe assoziiert ist, entspricht, wobei 2ab42 bestimmt wird, indem die Menge an ABETA42-Peptid in der Probe quantifiziert wird, nachdem die Probe mit einem Proteinsolubilisierungsmittel unter Bedingungen in Kontakt gebracht worden ist, die geeignet sind, um die Dissoziation des ABETA42-Peptids von den Bestandteilen, die in der biologischen Probe vorhanden sind, zu begünstigen,

(e) 3ab40, das der Konzentration von ABETA40-Peptid, das mit Zellen in einer biologischen Probe von dem Subjekt assoziiert ist, entspricht, wobei 3ab40 bestimmt wird, indem die Menge an ABETA40-Peptid quantifiziert wird, nachdem die zelluläre Fraktion der biologischen Probe mit einem Proteinsolubilisierungsmittel unter Bedingungen in Kontakt gebracht worden ist, die geeignet sind, um die Dissoziation der Amyloid-beta-Peptide von den Zellen, die in der Probe vorhanden sind, zu begünstigen, und

(f) 3ab42, das der Konzentration von ABETA42-Peptid, das mit Zellen in einer biologischen Probe von dem Subjekt assoziiert ist, entspricht, wobei 3ab42 bestimmt wird, indem die Menge an ABETA42-Peptid quantifiziert wird, nachdem die zelluläre Fraktion der biologischen Probe mit einem Proteinsolubilisierungsmittel unter Bedingungen in Kontakt gebracht worden ist, die geeignet sind, um die Dissoziation der Amyloid-beta-Peptide von den Zellen, die in der Probe vorhanden sind, zu begünstigen.

2. Verfahren nach Anspruch 1, wobei der berechnete Parameter, der in dem Schritt (ii) erhalten wird, aus der Gruppe aus 2ab40/2ab42, 3ab40/3ab42, 2ab40/3ab40, 2ab42/3ab42, 1ab40 + 2ab40, 1ab40 + 3ab40, 2ab40 + 3ab40, 1ab40 + 2ab40 + 3ab40, 1ab42 + 2ab42, 1ab42 + 3ab42, 2ab42 + 3ab42, 1 ab42 + 2ab42 + 3ab42, 1ab40 + 2ab40 + 1 ab42 + 2ab42, 1ab40 + 3ab40 + 1ab42 + 3ab42, 2ab40 + 3ab40 + 2ab42 + 3ab42, 1ab40 + 2ab40 + 3ab40 + 1ab42 + 2ab42 + 3ab42, (1ab40 + 2ab40)/(1ab42 + 2ab42), (1ab40 + 3ab40)/(1ab42 + 3ab42), (2ab40 + 3ab40)/(2ab42 + 3ab42), (1ab40 + 2ab40 + 3ab40)/(1ab42 + 2ab42 + 3ab42), (1ab42 + 2ab42)/(1ab40 + 2ab40), (1ab42 + 3ab42)/(1ab40 + 3ab40), (2ab42 + 3ab42)/(2ab40 + 3ab40), (1ab42 + 2ab42 + 3ab42)/(1ab40 + 2ab40 + 3ab40), 2ab40 - 1ab40, 2ab42 - 1 ab42 und (2ab40 - 1ab40)/(2ab42 - 1ab42) ausgewählt ist.

3. Verfahren nach Anspruch 1 oder 2, wobei

(i) die Diagnose einer neurodegenerativen Erkrankung durchgeführt wird, indem der Wert eines Parameters, der aus der Gruppe aus 3ab40, 2ab42 und 3ab42 ausgewählt ist, oder der Wert eines berechneten Parameters, der aus der Gruppe aus 2ab40 + 3ab40, 2ab42 + 3ab42, 1ab40 + 2ab40 + 1ab42 + 2ab42 und 2ab40 + 3ab40 + 2ab42 + 3ab42 ausgewählt ist, verglichen wird,

(ii) der Nachweis eines Stadiums vor einer neurodegenerativen Erkrankung durchgeführt wird, indem der Wert eines Parameters, der aus der Gruppe aus 2ab40, 3ab40, 2ab42 und 3ab42 ausgewählt ist, oder der Wert eines berechneten Parameters, der aus der Gruppe aus 2ab40 + 3ab40, 2ab42 + 3ab42, 2ab40 + 3ab40 + 2ab42 + 3ab42, 1ab40 + 2ab40 + 3ab40 + 1ab42 + 2ab42 + 3ab42, 1ab40 + 2ab40 + 3ab40, 1ab42 + 2ab42 + 3ab42, 1ab40 + 2ab40 + 1 ab42 + 2ab42 und 1ab40 + 3ab40 + 1 ab42 + 3ab42 ausgewählt ist, verglichen wird, oder

(iii) das Unterscheiden einer neurodegenerativen Erkrankung von einem Stadium vor der neurodegenerativen Erkrankung durchgeführt wird, indem der Wert eines Parameters, der aus der Gruppe aus 3ab40 und 2ab42 ausgewählt ist, oder der Wert eines berechneten Parameters, der aus der Gruppe aus 2ab40 + 3ab40, 2ab40 + 3ab40 + 2ab42 + 3ab42 und 1ab40 + 2ab40 + 1ab42 + 2ab42 ausgewählt ist, verglichen wird.

4. Verfahren nach Anspruch 3, wobei der Bezugswert, der in dem Schritt (ii) verwendet wird, um den Wert des Parameters oder den Wert des berechneten Parameters zu verglichen, aus der Gruppe ausgewählt ist, bestehend aus

(i) 63,8 pg/ml, wenn der Parameter 2ab40 für den Nachweis eines Stadiums vor einer neurodegenerativen Erkrankung verwendet wird,

(ii) 71,9 pg/ml, wenn der Parameter 3ab40 für die Diagnose einer neurodegenerativen Erkrankung verwendet wird,

(iii) 71,1 pg/ml, wenn der Parameter 3ab40 für den Nachweis eines Stadiums vor einer neurodegenerativen Erkrankung verwendet wird,

(iv) 211,3 pg/ml, wenn der Parameter 3ab40 für das Unterscheiden einer neurodegenerativen Erkrankung von einem Stadium vor der neurodegenerativen Erkrankung verwendet wird,

(v) 47,4 pg/ml, wenn der Parameter 2ab42 für die Diagnose einer neurodegenerativen Erkrankung verwendet wird,

(vi) 50,3 pg/ml, wenn der Parameter 2ab42 für den Nachweis eines Stadiums vor einer neurodegenerativen Erkrankung verwendet wird,

(vii) 151,7 pg/ml, wenn der Parameter 2ab42 für das Unterscheiden einer neurodegenerativen Erkrankung von einem Stadium vor der neurodegenerativen Erkrankung verwendet wird,

(viii) 76,9 pg/ml, wenn der Parameter 3ab42 ist und für die Diagnose einer neurodegenerativen Erkrankung verwendet wird,

(ix) 58,8 pg/ml, wenn der Parameter 3ab42 ist und für den Nachweis eines Stadiums vor einer neurodegenerativen Erkrankung verwendet wird,

(x) 132,7 pg/ml, wenn der Parameter 2ab40 + 3ab40 für die Diagnose einer neurodegenerativen Erkrankung verwendet wird,

(xi) 132,7 pg/ml, wenn der Parameter 2ab40 + 3ab40 für den Nachweis eines Stadiums vor einer neurodegenerativen Erkrankung verwendet wird,

(xii) 550,8 pg/ml, wenn der Parameter 2ab40 + 3ab40 für das Unterscheiden einer neurodegenerativen Erkrankung von einem Stadium vor der neurodegenerativen Erkrankung verwendet wird,

(xiii) 115,8 pg/ml, wenn der Parameter 2ab42 + 3ab42 ist und für die Diagnose einer neurodegenerativen Erkrankung verwendet wird,

(xiv) 103,3 pg/ml, wenn der Parameter 2ab42 + 3ab42 ist und für den Nachweis eines Stadiums vor einer neurodegenerativen Erkrankung verwendet wird,

(xv) 235,5 pg/ml, wenn der Parameter 2ab40 + 3ab40 + 2ab42 + 3ab42 für die Diagnose einer neurodegenerativen Erkrankung verwendet wird,

(xvi) 235,5 pg/ml, wenn der Parameter 2ab40 + 3ab40 + 2ab42 + 3ab42 für den Nachweis eines Stadiums vor einer neurodegenerativen Erkrankung verwendet wird,

(xvii) 778,1 pg/ml, wenn der Parameter 2ab40 + 3ab40 + 2ab42 + 3ab42 für das Unterscheiden einer neurodegenerativen Erkrankung von einem Stadium vor der neurodegenerativen Erkrankung verwendet wird,

(xviii) 272,1 pg/ml, wenn der Parameter 1ab40 + 2ab40 + 3ab40 + 1ab42 + 2ab42 + 3ab42 für den Nachweis eines Stadiums vor einer neurodegenerativen Erkrankung verwendet wird,

(xix) 155,8 pg/ml, wenn der Parameter 1ab40 + 2ab40 + 3ab40 für den Nachweis eines Stadiums vor einer neurodegenerativen Erkrankung verwendet wird,

(xx) 124,3 pg/ml, wenn der Parameter 1ab42 + 2ab42 + 3ab42 für den Nachweis eines Stadiums vor einer neurodegenerativen Erkrankung verwendet wird,

(xxi) 158,3 pg/ml, wenn der Parameter 1ab40 + 2ab40 + 1ab42 + 2ab42 für die Diagnose einer neurodegenerativen Erkrankung verwendet wird,

(xxii) 142,4 pg/ml, wenn der Parameter 1ab40 + 2ab40 + 1ab42 + 2ab42 für den Nachweis eines Stadiums vor einer neurodegenerativen Erkrankung verwendet wird,

(xxiii) 161,2 pg/ml, wenn der Parameter 1ab40 + 2ab40 + 1ab42 + 2ab42 für das Unterscheiden einer neurodegenerativen Erkrankung von einem Stadium vor der neurodegenerativen Erkrankung verwendet wird, und

(xxiv) 154,7 pg/ml, wenn der Parameter 1ab40 + 3ab40 + 1ab42 + 3ab42 für den Nachweis eines Stadiums vor einer neurodegenerativen Erkrankung verwendet wird.

5. Verfahren nach einem der Ansprüche 1 bis 4, wobei die Veränderung in dem Wert des Parameters oder in dem Wert des berechneten Parameters in Bezug auf den Bezugswert eine Zunahme ist.

6. Verfahren nach einem der Ansprüche 1 bis 5, wobei das Proteinsolubilisierungsmittel ein Detergens, vorzugsweise Tween 20, ist.

7. Verfahren nach einem der Ansprüche 1 bis 6, wobei der Bestimmungsschritt von dem mindestens einen oder mehreren aus 1ab40, 1ab42, 2ab40, 2ab42, 3ab40 und 3ab42 durch ein immunologisches Verfahren durchgeführt wird.

8. Verfahren nach Anspruch 7, wobei das immunologische Verfahren ein ELISA-Assay ist.

9. Verfahren nach Anspruch 8, wobei der ELISA-Assay ein ELISA-Sandwich-Assay ist.

10. Verfahren nach Anspruch 9, wobei der Fänger-Antikörper in dem ELISA-Sandwich-Assay ein Antikörper gegen die N-terminale Region des Amyloid-beta-Peptids ist.

11. Verfahren nach Anspruch 10, wobei der Antikörper gegen die N-terminale Region des Amyloid-beta-Peptids gegen

# EP 2 510 359 B1

ein Epitop gerichtet ist, das innerhalb der Aminosäuren 1 bis 7 des ABETA40 und ABETA42 lokalisiert ist.

**12.** Verfahren nach einem der Ansprüche 8 bis 11, wobei der Nachweis-Antikörper in dem ELISA-Sandwich-Assay ein Antikörper ist, der spezifisch gegen ein Epitop ist, das in der C-terminalen Region des Amyloid-beta-Peptids lokalisiert ist.

**13.** Verfahren nach Anspruch 12, wobei der Antikörper, der spezifisch gegen die C-terminale Region des Amyloid-beta-Peptids ist, aus der Gruppe ausgewählt ist, bestehend aus

(i) einem polyklonalen Antikörper, der gegen ein Peptid hergestellt ist, das der C-terminalen Region des ABETA42-Peptids entspricht, und der spezifisch an ABETA42 bindet, ohne irgendeine wesentliche Kreuzreaktion mit ABETA40 zu ergeben,
(ii) einem polyklonalen Antikörper, der gegen ein Peptid hergestellt ist, das der C-terminalen Region des ABETA40-Peptids entspricht, und der spezifisch an ABETA40 bindet, ohne irgendeine wesentliche Kreuzreaktion mit ABETA42 zu ergeben, und
(iii) einem Antikörper, der gleichzeitig die C-terminale Region von sowohl ABETA40 als auch ABETA42 erkennt, und
(iv) einer Kombination aus den Antikörpern aus (i) und (ii).

**14.** Verfahren nach einem der Ansprüche 9 bis 13, wobei der Nachweis-Antikörper ferner unter Verwendung eines Reagens nachgewiesen wird, das eine Affinität für den Antikörper aufweist, der an ein erstes Element eines Bindungspaars gekoppelt ist.

**15.** Verfahren nach Anspruch 14, wobei der Nachweis unter Verwendung eines zweiten Elements eines Bindungspaars, das an ein nachweisbares Tag gekoppelt ist, durchgeführt wird.

**16.** Verfahren nach einem der Ansprüche 1 bis 15, wobei die neurodegenerative Erkrankung eine Alzheimer-Krankheit und/oder das Stadium vor einer neurodegenerativen Erkrankung eine milde kognitive Beeinträchtigung ist.

## Revendications

**1.** Procédé pour le diagnostic d'une maladie neurodégénérative chez un sujet, pour détecter une phase avant une maladie neurodégénérative ou pour distinguer une maladie neurodégénérative à partir d'une phase antérieure à ladite maladie neurodégénérative, comprenant les étapes consistant à :

(i) déterminer un ou plusieurs paramètres choisis dans le groupe constitué :

(a) du niveau d'un ou plusieurs peptides bêta-amyloïdes libres dans un échantillon biologique dudit sujet,
(b) des niveaux groupés d'un ou plusieurs peptides amyloïdes libres dans un échantillon biologique dudit sujet et dudit un ou plusieurs peptides bêta-amyloïdes associés aux composants macromoléculaires présents dans ledit échantillon biologique, dans lequel lesdits niveaux groupés sont déterminés en quantifiant la quantité dudit un ou plusieurs peptides bêta-amyloïdes dans une fraction sans cellule dudit échantillon, après l'entrée en contact dudit échantillon avec un agent de solubilisation de protéine dans des conditions adéquates pour promouvoir une dissociation du ou des peptide(s) bêta-amyloïde(s) à partir des composants présents dans l'échantillon biologique,
(c) du niveau d'un ou plusieurs peptides bêta-amyloïdes associés aux cellules dans un échantillon biologique dudit sujet, dans lequel ledit niveau est déterminé en isolant la fraction cellulaire dudit échantillon biologique, en mettant en contact ladite fraction cellulaire dudit échantillon avec un agent de solubilisation de protéine dans des conditions adéquates pour promouvoir la dissociation du ou des peptide(s) bêta-amyloïde(s) des cellules présentes dans l'échantillon et

(ii) comparer la valeur d'au moins un des paramètres (b) ou (c) ou la valeur d'un paramètre calculé résultant d'une combinaison arithmétique d'au moins deux des paramètres (a) à (c), avec une valeur de référence correspondant à la valeur desdits paramètres (b) ou (c) ou dudit paramètre calculé dans un échantillon de référence et
(iii) diagnostiquer la maladie neurodégénérative, détecter une phase antérieure à une maladie neurodégénérative, ou distinguer une maladie neurodégénérative à partir d'une phase antérieure à ladite maladie neurodégénérative, quand il existe une altération de la valeur du paramètre ou de la valeur du paramètre calculé, relativement à la valeur de référence,

dans lequel l'échantillon biologique est un échantillon de plasma ou de sang, et dans lequel les paramètres déterminés à l'étape (i) sont un ou plusieurs des paramètres choisis dans le groupe constitué de :

(a) 1ab40, correspondant au niveau de peptide ABETA40 libre dans un échantillon biologique dudit sujet,

(b) 1ab42, correspondant au niveau de peptide ABETA42 libre dans un échantillon biologique dudit sujet,

(c) 2ab40, correspondant aux niveaux groupés de peptide ABETA40 libre dans un échantillon biologique dudit sujet et de peptide ABETA40 associé aux composants dudit échantillon biologique, dans lequel 2ab40 est déterminé en quantifiant la quantité de peptide ABETA40 dans ledit échantillon après la mise en contact dudit échantillon avec un agent de solubilisation de protéine, dans des conditions adéquates pour promouvoir la dissociation du peptide ABETA40 des composants présents dans l'échantillon biologique.

(d) 2ab42, correspondant au niveau groupé de peptide ABETA42 libre dans un échantillon biologique dudit sujet et de peptide ABETA42 associé aux composants dudit échantillon biologique, dans lequel 2ab42 est déterminé en quantifiant la quantité de peptide ABETA42 dans ledit échantillon après mise en contact dudit échantillon avec un agent solubilisant de protéine, dans des conditions adéquates pour promouvoir la dissociation du peptide ABETA42 des composants présents dans l'échantillon biologique,

(e) 3ab40, correspondant au niveau du peptide ABETA40 associé aux cellules dans un échantillon biologique dudit sujet, dans lequel le 3ab40 est déterminé en quantifiant la quantité de peptide ABETA40 après mise en contact de la fraction cellulaire dudit échantillon biologique avec un agent de solubilisation de protéine, dans des conditions adéquates pour promouvoir la dissociation des peptides bêta-amyloïdes des cellules présentes dans l'échantillon et

(f) 3ab42, correspondant au niveau du peptide ABETA42 associé aux cellules dans un échantillon biologique dudit sujet, dans lequel le 3ab42 est déterminé en quantifiant la quantité de peptide ABETA42 après mise en contact de la fraction cellulaire dudit échantillon biologique avec un agent de solubilisation de protéine, dans des conditions adéquates pour promouvoir la dissociation des peptides bêta-amyloïdes des cellules présentes dans l'échantillon.

2. Procédé selon la revendication 1, dans lequel le paramètre calculé obtenu dans l'étape (ii) est choisi dans le groupe de 2ab40/2ab42, 3ab40/3ab42, 2ab40/3ab40, 2ab42/3ab42, 1ab40 + 2 ab40, 1ab40 + 3ab40, 2ab40 + 3ab40, 1ab40 + 2ab40 + 3ab40, 1ab42 + 2ab42, 1ab42 + 3ab42, 2ab42 + 3ab42, 1ab42 + 2ab42 + 3ab42, 1ab40 + 2ab40 + 1ab42 + 2ab42, 1ab40 + 3ab40 + 1ab42 + 3ab42, 2ab40 + 3ab40 + 2ab42 + 3ab42, 1ab40 + 2ab40 + 3ab40 + 1ab42 + 2ab42 + 3ab42, (1ab40 + 2ab40)/(1ab42 + 2ab42), (1ab40 + 3ab40)/(1ab42 + 3ab42), (2ab40 + 3ab40)/(2ab42 + 3ab42), (1ab40 + 2ab40 + 3ab40)/(1ab42 + 2ab42 + 3ab42), (1ab42 + 2ab42)/(1ab40 + 2ab40), (1ab42 + 3ab42)/(1ab40 + 3ab40), (2ab42 + 3ab42)/(2ab40 + 3ab40), (1ab42 + 2ab42 + 3ab42)/(1ab40 + 2ab40 + 3ab40), 2ab40 - 1ab40, 2ab42 - 1ab42 et (2ab40-1ab40)/(2ab42 - 1ab42).

3. Procédé selon les revendications 1 ou 2, dans lequel

(i) le diagnostic d'une maladie neurodégénérative est effectué en comparant la valeur d'un paramètre choisi dans le groupe de 3ab40, 2ab42 et 3ab42 ou la valeur d'un paramètre calculé choisi dans le groupe de 2ab40 + 3ab40, 2ab42 + 3ab42, 1ab40 + 2ab40 + 1ab42 + 2ab42 et 2ab40 + 3ab40 + 2ab42 + 3ab42,

(ii) la détection d'une phase antérieure à une maladie neurodégénérative est effectuée en comparant la valeur d'un paramètre choisi dans le groupe constitué de 2ab40, 3ab40, 2ab42 et 3ab42 ou la valeur d'un paramètre calculé choisi dans le groupe constitué de 2ab40 + 3ab40, 2ab42 + 3ab42, 2ab40 + 3ab40 + 2ab42 + 3ab42, 1ab40 + 2ab40 + 3ab40 + 1ab42 + 2ab42 + 3ab42, 1ab40 + 2ab40 + 3ab40, 1ab42 + 2ab42 + 3ab42, 1ab40 + 2ab40 + 1ab42 + 2ab42 et 1ab40 + 3ab40 + 1ab42 + 3ab42 ou

(iii) la distinction d'une maladie neurodégénérative à partir d'une phase antérieure à ladite maladie neurodégénérative est réalisée en comparant la valeur d'un paramètre choisi dans le groupe de 3ab40 et 2ab42 ou en comparant la valeur d'un paramètre calculé choisi dans le groupe de 2ab40 + 3ab40, 2ab40 + 3ab40 + 2ab42 + 3ab42 et 1ab40 + 2ab40 + 1ab42 + 2ab42.

4. Procédé selon la revendication 3, dans lequel la valeur de référence utilisée dans l'étape (ii) pour comparer la valeur du paramètre ou la valeur du paramètre calculé est choisie dans le groupe de

(i) 63,8 pg/ml quand le paramètre 2ab40 est utilisé pour la détection d'une phase antérieure à une maladie neurodégénérative ;

(ii) 71,9 pg/ml quand le paramètre 3ab40 est utilisé pour le diagnostic d'une maladie neurodégénérative,

(iii) 71,1 pg/ml quand le paramètre 3ab40 est utilisé pour la détection d'une phase antérieure à une maladie neurodégénérative ;

(iv) 211,3 pg/ml quand le paramètre 3ab40 est utilisé pour distinguer une maladie neurodégénérative à partir d'une phase antérieure à ladite maladie neurodégénérative ;

(v) 47,4 pg/ml quand le paramètre 2ab42 est utilisé pour le diagnostic d'une maladie neurodégénérative,

(vi) 50,3 pg/ml quand le paramètre 2ab42 est utilisé pour la détection d'une phase antérieure à une maladie neurodégénérative ;

(vii) 151,7 pg/ml quand le paramètre 2ab42 est utilisé pour distinguer une maladie neurodégénérative à partir d'une phase antérieure à ladite maladie neurodégénérative ;

(viii) 76,9 pg/ml quand le paramètre est 3ab42 et est utilisé pour le diagnostic d'une maladie neurodégénérative ;

(ix) 58,8 pg/ml quand le paramètre est 3ab42 et est utilisé pour la détection d'une phase antérieure à une maladie neurodégénérative ;

(x) 132,7 pg/ml quand le paramètre 2ab40 + 3ab40 est utilisé pour le diagnostic d'une maladie neurodégénérative ;

(xi) 132,7 pg/ml quand le paramètre 2ab40 + 3ab40 est utilisé pour la détection d'une phase antérieure à une maladie neurodégénérative ;

(xii) 550,8 pg/ml quand le paramètre 2ab40 + 3ab40 est utilisé pour distinguer une maladie neurodégénérative à partir d'une phase antérieure à ladite maladie neurodégénérative ;

(xiii) 115,8 pg/ml quand le paramètre est 2ab42 + 3ab42 et est utilisé pour le diagnostic d'une maladie neurodégénérative ;

(xiv) 103,3 pg/ml quand le paramètre est 2ab42 + 3ab42 et est utilisé pour la détection d'une phase antérieure à une maladie neurodégénérative ;

(xv) 235,5 pg/ml quand le paramètre 2ab40 + 3ab40 + 2ab42 + 3ab42 est utilisé pour le diagnostic d'une maladie neurodégénérative ;

(xvi) 235,5 pg/ml quand le paramètre 2ab40 + 3ab40 + 2ab42 + 3ab42 est utilisé pour la détection d'une phase antérieure à une maladie neurodégénérative ;

(xvii) 778,1 pg/ml quand le paramètre 2ab40 + 3ab40 + 2ab42 + 3ab42 est utilisé pour distinguer une maladie neurodégénérative à partir d'une phase antérieure à ladite maladie neurodégénérative ;

(xviii) 272,1 pg/ml quand le paramètre 1ab40 + 2ab40 + 3ab40 + 1ab42 + 2ab42 + 3ab42 est utilisé pour la détection d'une phase antérieure à une maladie neurodégénérative,

(xix) 155,8 pg/ml quand le paramètre 1ab40 + 2ab40 + 3ab40 est utilisé pour la détection d'une phase antérieure à une maladie neurodégénérative,

(xx) 124,3 pg/ml quand le paramètre 1ab42 + 2ab42 + 3ab42 est utilisé pour la détection d'une phase antérieure à une maladie neurodégénérative,

(xxi) 158,3 pg/ml quand le paramètre 1ab40 + 2ab40 + 1ab42 + 2ab42 est utilisé pour le diagnostic d'une maladie neurodégénérative,

(xxii) 142,4 pg/ml quand le paramètre 1ab40 + 2ab40 + 1ab42 + 2ab42 est utilisé pour la détection d'une phase antérieure à une maladie neurodégénérative,

(xxiii) 161,2 pg/ml quand le paramètre 1ab40 + 2ab40 + 1ab42 + 2ab42 est utilisé pour distinguer une maladie neurodégénérative à partir d'une phase antérieure à ladite maladie neurodégénérative et

(xxiv) 154,7 pg/ml quand le paramètre 1ab40 + 3ab40 + 1ab42 + 3ab42 est utilisé pour la détection d'une phase antérieure à une maladie neurodégénérative.

5. Procédé selon l'une quelconque des revendications 1 à 4, dans lequel l'altération de la valeur du paramètre et de la valeur du paramètre calculé relativement à la valeur de référence est une augmentation.

6. Procédé selon l'une quelconque des revendications 1 à 5, dans lequel l'agent de solubilisation de protéine est un détergent, de préférence Tween 20.

7. Procédé selon l'une quelconque des revendications 1 à 6, dans lequel l'étape de détermination dudit au moins un ou plusieurs éléments parmi 1ab40, 1ab42, 2ab40, 2ab42, 3ab40 et 3ab42 est réalisée par un procédé immunologique.

8. Procédé selon la revendication 7, dans lequel ledit procédé immunologique est un test ELISA.

9. Procédé selon la revendication 8, dans lequel l'essai ELISA est un test ELISA « en sandwich ».

**10.** Procédé selon la revendication 9, dans lequel l'anticorps de capture dans le test ELISA en sandwich est un anticorps contre la zone N-terminale du peptide bêta-amyloïde.

**11.** Procédé selon la revendication 10, dans lequel l'anticorps contre la zone N-terminale du peptide bêta-amyloïde est dirigé contre un épitope situé dans des acides aminés 1 à 7 d'ABETA40 et ABETA42.

**12.** Procédé selon l'une quelconque des revendications 8 à 11, dans lequel l'anticorps de détection dans le test ELISA en sandwich est un anticorps spécifique contre un épitope situé dans la zone C-terminale du peptide bêta-amyloïde.

**13.** Procédé selon la revendication 12, dans lequel l'anticorps spécifique contre la zone C-terminale du peptide bêta-amyloïde est choisi dans le groupe constitué de :

(i) un anticorps polyclonal préparé contre un peptide correspondant à la zone C-terminale du peptide ABETA42 qui se lie spécifiquement à ABETA42 sans donner de réaction croisée substantielle avec ABETA40,
(ii) un anticorps polyclonal préparé contre un peptide correspondant à la zone C-terminale du peptide ABETA40 qui se lie spécifiquement à ABETA40 sans donner de réaction croisée substantielle avec ABETA42 et
(iii) un anticorps qui reconnaît simultanément la zone C-terminale d'ABETA40 et d'ABETA42, et (iv) une combinaison d'anticorps aux termes de (i) et (ii).

**14.** Procédé selon l'une quelconque des revendications 9 à 13, dans lequel l'anticorps de détection est en outre détecté en utilisant un réactif présentant une affinité pour ledit anticorps, qui est couplé à un premier élément d'une paire de liaison.

**15.** Procédé selon la revendication 14, dans lequel la détection est réalisée en utilisant un second élément d'une paire de liaison couplée à un marqueur détectable.

**16.** Procédé selon l'une quelconque des revendications 1 à 15, dans lequel la maladie neurodégénérative est la maladie d'Alzheimer et/ou la phase antérieure à une maladie neurodégénérative est une altération cognitive légère.

FIGURE 1

FIGURE 2

FIGURE 3

FIGURE 4

FIGURE 5

54

FIGURE 6

FIGURE 7

FIGURE 8

FIGURE 9

56

FIGURE 10

FIGURE 11

FIGURE 12

FIGURE 13

FIGURE 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200722015 A
- WO 200750359 A
- WO 0162801 A
- WO 0315617 A
- WO 0246237 A
- WO 0413172 A
- WO 200646644 A
- WO 2009015696 A
- WO 2006053251 A
- WO 2004024770 A
- WO 2004098631 A
- EP 09382279 A

**Non-patent literature cited in the description**

- **SCHEUNER et al.** *Nature Med.,* 1996, vol. 2, 864-870
- **TAMAOKA A et al.** *J Neurol Sci.,* 1996, vol. 141, 65-68
- **SUZUKI,N. et al.** *Science,* 1994, vol. 264, 1336-1340
- **VANDERSTICHELE H et al.** *Amyloid,* 2000, vol. 7, 245-258
- **FUKOMOTO.** *Arch. Neurol.,* 2003, vol. 60, 958-964
- **MEHTA et al.** *Arch. Neurol.,* 2000, vol. 57, 100-105
- **MAYEUX, R. et al.** *Ann Neurol.,* 1999, vol. 46, 412-416
- **LANZ, T.A ; SCHACTHTER, J.B.** *J. Neuroscience Methods,* 2006, vol. 157, 71-81
- **DOWDY ; WEARDEN.** Statistics for Research. John Wiley & Sons, 1983
- **SAMBROOK et al.** Molecular Cloning: A Laboratory Manual. Cold Spring Harbor Laboratory Press, 1989
- MOLECULAR CLONING: A LABORATORY MANUAL. 2001
- **KOHLER et al.** *Nature,* vol. 256, 495
- **AUSUBEL, F.M. et al.** Current Protocols in Molecular Biology. John Wiley & Sons Inc, 2003
- **MCCAFFERTY et al.** *Nature,* 1990, vol. 348, 552-554
- **CLACKSOII et al.** *Nature,* 1991, vol. 352, 624-628
- **MARKS et al.** *J. Mol. Biol.,* 1991, vol. 222, 581-597
- **MARKS et al.** *Bio/Technology,* 1992, vol. 10, 779-783
- **WATERHOUSE et al.** *Nucl. Acids. Res.,* 1993, vol. 21, 2265-2266
- **KIM, K.S.** *Neuroscience Res. Comm.,* 1988, vol. 2, 121-130
- **SCHELTENS.** *J. Neurol. Neurosurg Psychiatry,* 1992, vol. 55, 967-972
- **FUKUMOTO et al.** *Arch. Neurol.,* 2003, vol. 60, 958-964

SEQUENCE LISTING

<110> ARACLON BIOTECH S.L.

<120> METHODS AND REAGENTS FOR IMPROVED DETECTION OF AMYLOID BETA PEPTIDES

<130> P4599PC00

<150> EP09382279
<151> 2009-12-11

<160> 15

<170> Patent In version 3.5

<210> 1
<211> 40
<212> PRT
<213> Homo sapiens

<400> 1

```
Asp Ala Glu Phe Arg His Asp Ser Gly Tyr Glu Val His His Gln Lys
1               5               10                  15

Leu Val Phe Phe Ala Glu Asp Val Gly Ser Asn Lys Gly Ala Ile Ile
            20              25              30

Gly Leu Met Val Gly Gly Val Val
        35              40
```

<210> 2
<211> 38
<212> PRT
<213> Homo sapiens

<400> 2

```
        Asp Ala Glu Phe Arg His Asp Ser Gly Tyr Glu Val His His Gln Lys
        1               5               10              15

        Leu Val Phe Phe Ala Glu Asp Val Gly Ser Asn Lys Gly Ala Ile Ile
                    20              25              30

        Gly Leu Met Val Gly Gly
                    35
```

<210> 3
<211> 39
<212> PRT
<213> Homo sapiens

<400> 3

```
        Asp Ala Glu Phe Arg His Asp Ser Gly Tyr Glu Val His His Gln Lys
        1               5               10              15

        Leu Val Phe Phe Ala Glu Asp Val Gly Ser Asn Lys Gly Ala Ile Ile
                    20              25              30

        Gly Leu Met Val Gly Gly Val
                    35
```

<210> 4
<211> 41
<212> PRT
<213> Homo sapiens

<400> 4

```
        Asp Ala Glu Phe Arg His Asp Ser Gly Tyr Glu Val His His Gln Lys
        1               5               10              15

        Leu Val Phe Phe Ala Glu Asp Val Gly Ser Asn Lys Gly Ala Ile Ile
                    20              25              30

        Gly Leu Met Val Gly Gly Val Val Ile
                    35                  40
```

<210> 5
<211> 42
<212> PRT
<213> Homo sapiens

<400> 5

```
        Asp Ala Glu Phe Arg His Asp Ser Gly Tyr Glu Val His His Gln Lys
        1               5               10              15

        Leu Val Phe Phe Ala Glu Asp Val Gly Ser Asn Lys Gly Ala Ile Ile
                    20              25                  30

        Gly Leu Met Val Gly Gly Val Val Ile Ala
                    35              40
```

<210> 6
<211> 43
<212> PRT
<213> Homo sapiens

<400> 6

```
        Asp Ala Glu Phe Arg His Asp Ser Gly Tyr Glu Val His His Gln Lys
        1               5               10              15

        Leu Val Phe Phe Ala Glu Asp Val Gly Ser Asn Lys Gly Ala Ile Ile
                    20              25                  30

        Gly Leu Met Val Gly Gly Val Val Ile Ala Thr
                    35              40
```

<210> 7
<211> 32
<212> PRT
<213> Homo sapiens

<400> 7

```
        Glu Val His His Gln Lys Leu Val Phe Phe Ala Glu Asp Val Gly Ser
        1               5               10              15

        Asn Lys Gly Ala Ile Ile Gly Leu Met Val Gly Gly Val Val Ile Ala
                    20              25                  30
```

<210> 8
<211> 38
<212> PRT
<213> Homo sapiens

<400> 8

```
Glu Phe Arg His Asp Ser Gly Tyr Glu Val His His Gln Lys Leu Val
1               5                   10              15

Phe Phe Ala Glu Asp Val Gly Ser Asn Lys Gly Ala Ile Ile Gly Leu
            20                  25                  30

Met Val Gly Gly Val Val
            35
```

<210> 9
<211> 40
<212> PRT
<213> Homo sapiens

<400> 9

```
Glu Phe Arg His Asp Ser Gly Tyr Glu Val His His Gln Lys Leu Val
1               5                   10              15

Phe Phe Ala Glu Asp Val Gly Ser Asn Lys Gly Ala Ile Ile Gly Leu
            20                  25                  30

Met Val Gly Gly Val Val Ile Ala
            35                  40
```

<210> 10
<211> 39
<212> PRT
<213> Homo sapiens

<400> 10

```
Phe Arg His Asp Ser Gly Tyr Glu Val His His Gln Lys Leu Val Phe
1               5                   10              15

Phe Ala Glu Asp Val Gly Ser Asn Lys Gly Ala Ile Ile Gly Leu Met

                20                  25                  30

Val Gly Gly Val Val Ile Ala
            35
```

<210> 11
<211> 37
<212> PRT
<213> Homo sapiens

<400> 11

```
His Asp Ser Gly Tyr Glu Val His His Gln Lys Leu Val Phe Phe Ala
1               5                   10              15

Glu Asp Val Gly Ser Asn Lys Gly Ala Ile Ile Gly Leu Met Val Gly
                20                  25              30

Gly Val Val Ile Ala
            35
```

<210> 12
<211> 36
<212> PRT
<213> Homo sapiens

<400> 12

```
Asp Ser Gly Tyr Glu Val His His Gln Lys Leu Val Phe Phe Ala Glu
1               5                   10              15

Asp Val Gly Ser Asn Lys Gly Ala Ile Ile Gly Leu Met Val Gly Gly
            20                  25              30

Val Val Ile Ala
        35
```

<210> 13
<211> 35
<212> PRT
<213> Homo sapiens

<400> 13

```
Ser Gly Tyr Glu Val His His Gln Lys Leu Val Phe Phe Ala Glu Asp
1               5                   10              15

Val Gly Ser Asn Lys Gly Ala Ile Ile Gly Leu Met Val Gly Gly Val
            20                  25              30

Val Ile Ala
        35
```

<210> 14
<211> 34
<212> PRT
<213> Homo sapiens

<400> 14

```
Gly Tyr Glu Val His His Gln Lys Leu Val Phe Phe Ala Glu Asp Val
1               5                   10                  15


Gly Ser Asn Lys Gly Ala Ile Ile Gly Leu Met Val Gly Gly Val Val
            20                  25                  30


Ile Ala
```

<210> 15
<211> 770
<212> PRT
<213> Homo sapiens

<400> 15

```
Met Leu Pro Gly Leu Ala Leu Leu Leu Leu Ala Ala Trp Thr Ala Arg
1               5                   10                  15


Ala Leu Glu Val Pro Thr Asp Gly Asn Ala Gly Leu Leu Ala Glu Pro
            20                  25                  30


Gln Ile Ala Met Phe Cys Gly Arg Leu Asn Met His Met Asn Val Gln
        35                  40                  45


Asn Gly Lys Trp Asp Ser Asp Pro Ser Gly Thr Lys Thr Cys Ile Asp
        50                  55                  60


Thr Lys Glu Gly Ile Leu Gln Tyr Cys Gln Glu Val Tyr Pro Glu Leu
65                  70                  75                  80


Gln Ile Thr Asn Val Val Glu Ala Asn Gln Pro Val Thr Ile Gln Asn
                85                  90                  95


Trp Cys Lys Arg Gly Arg Lys Gln Cys Lys Thr His Pro His Phe Val
            100                 105                 110


Ile Pro Tyr Arg Cys Leu Val Gly Glu Phe Val Ser Asp Ala Leu Leu
        115                 120                 125


Val Pro Asp Lys Cys Lys Phe Leu His Gln Glu Arg Met Asp Val Cys
        130                 135                 140


Glu Thr His Leu His Trp His Thr Val Ala Lys Glu Thr Cys Ser Glu
145                 150                 155                 160
```

```
Lys Ser Thr Asn Leu His Asp Tyr Gly Met Leu Leu Pro Cys Gly Ile
            165             170             175

Asp Lys Phe Arg Gly Val Glu Phe Val Cys Cys Pro Leu Ala Glu Glu
            180             185             190

Ser Asp Asn Val Asp Ser Ala Asp Ala Glu Glu Asp Asp Ser Asp Val
            195             200             205

Trp Trp Gly Gly Ala Asp Thr Asp Tyr Ala Asp Gly Ser Glu Asp Lys
210             215             220

Val Val Glu Val Ala Glu Glu Glu Val Ala Glu Val Glu Glu Glu
225             230             235             240

Glu Ala Asp Asp Asp Glu Asp Asp Glu Asp Gly Asp Glu Val Glu Glu
            245             250             255

Glu Ala Glu Glu Pro Tyr Glu Glu Ala Thr Glu Arg Thr Thr Ser Ile
            260             265             270

Ala Thr Thr Thr Thr Thr Thr Glu Ser Val Glu Glu Val Val Arg
            275             280             285

Glu Val Cys Ser Glu Gln Ala Glu Thr Gly Pro Cys Arg Ala Met Ile
290             295             300

Ser Arg Trp Tyr Phe Asp Val Thr Glu Gly Lys Cys Ala Pro Phe Phe
305             310             315             320

Tyr Gly Gly Cys Gly Gly Asn Arg Asn Asn Phe Asp Thr Glu Glu Tyr
            325             330             335

Cys Met Ala Val Cys Gly Ser Ala Met Ser Gln Ser Leu Leu Lys Thr
            340             345             350

Thr Gln Glu Pro Leu Ala Arg Asp Pro Val Lys Leu Pro Thr Thr Ala
            355             360             365

Ala Ser Thr Pro Asp Ala Val Asp Lys Tyr Leu Glu Thr Pro Gly Asp
370             375             380

Glu Asn Glu His Ala His Phe Gln Lys Ala Lys Glu Arg Leu Glu Ala
385             390             395             400

Lys His Arg Glu Arg Met Ser Gln Val Met Arg Glu Trp Glu Glu Ala
            405             410             415

Glu Arg Gln Ala Lys Asn Leu Pro Lys Ala Asp Lys Lys Ala Val Ile
```

|  |  |  | 420 |  |  |  |  | 425 |  |  |  |  | 430 |  |  |
|---|---|---|---|---|---|---|---|---|---|---|---|---|---|---|---|

Gln His Phe Gln Glu Lys Val Glu Ser Leu Glu Gln Glu Ala Ala Asn
       435          440          445

Glu Arg Gln Gln Leu Val Glu Thr His Met Ala Arg Val Glu Ala Met
   450          455          460

Leu Asn Asp Arg Arg Arg Leu Ala Leu Glu Asn Tyr Ile Thr Ala Leu
465          470         475          480

Gln Ala Val Pro Pro Arg Pro Arg His Val Phe Asn Met Leu Lys Lys
       485          490          495

Tyr Val Arg Ala Glu Gln Lys Asp Arg Gln His Thr Leu Lys His Phe
      500          505          510

Glu His Val Arg Met Val Asp Pro Lys Lys Ala Ala Gln Ile Arg Ser
      515          520          525

Gln Val Met Thr His Leu Arg Val Ile Tyr Glu Arg Met Asn Gln Ser
      530          535          540

Leu Ser Leu Leu Tyr Asn Val Pro Ala Val Ala Glu Glu Ile Gln Asp
545          550         555          560

Glu Val Asp Glu Leu Leu Gln Lys Glu Gln Asn Tyr Ser Asp Asp Val
       565          570          575

Leu Ala Asn Met Ile Ser Glu Pro Arg Ile Ser Tyr Gly Asn Asp Ala
      580          585          590

Leu Met Pro Ser Leu Thr Glu Thr Lys Thr Thr Val Glu Leu Leu Pro
      595          600          605

Val Asn Gly Glu Phe Ser Leu Asp Asp Leu Gln Pro Trp His Ser Phe
      610          615          620

Gly Ala Asp Ser Val Pro Ala Asn Thr Glu Asn Glu Val Glu Pro Val
625          630         635          640

Asp Ala Arg Pro Ala Ala Asp Arg Gly Leu Thr Thr Arg Pro Gly Ser
       645          650          655

Gly Leu Thr Asn Ile Lys Thr Glu Glu Ile Ser Glu Val Lys Met Asp
      660          665          670

Ala Glu Phe Arg His Asp Ser Gly Tyr Glu Val His His Gln Lys Leu
      675          680          685

```
Val Phe Phe Ala Glu Asp Val Gly Ser Asn Lys Gly Ala Ile Ile Gly
    690                 695                 700

Leu Met Val Gly Gly Val Val Ile Ala Thr Val Ile Val Ile Thr Leu
705                 710                 715                 720

Val Met Leu Lys Lys Lys Gln Tyr Thr Ser Ile His His Gly Val Val
                725                 730                 735

Glu Val Asp Ala Ala Val Thr Pro Glu Glu Arg His Leu Ser Lys Met
            740                 745                 750

Gln Gln Asn Gly Tyr Glu Asn Pro Thr Tyr Lys Phe Phe Glu Gln Met
        755                 760                 765

Gln Asn
    770
```